# EUROPEAN PATENT APPLICATION

(11) **EP 3 901 618 A1**
(43) Date of publication of application: **27.10.2021**
(21) Application number: 21162422.6
(22) Date of filing: 12.03.2021
(51) Int. Cl.: G01N 21/65, G01J 3/44, A61B 5/00

(54) **METHOD AND APPARATUS FOR MEASURING SPECTRUM OF RAMAN-SCATTERED LIGHT USING TIME GATED DETECTION**

(30) Priority: 30.03.2020 FI 20205313
(71) Applicant: TimeGate Instruments Oy, 90590 Oulu (FI)
(72) Inventor: Kurki, Lauri, 90570 Oulu (FI); Tenhunen, Mari, 90910 Kontio (FI)
(74) Representative: Aaltonen, Janne Lari Antero

(57) **Abstract**

An apparatus (500) for measuring a spectrum (R_{M}(λ)) of Raman-scattered light (LB2). The apparatus comprises a light source (LS1) configured to provide illuminating light pulses (LB1), and an optical probe (100) to guide the illuminating light pulses to a sample region (REG1) and cause excitation of Raman-scattered light in the sample region. The optical probe comprises a waveguiding core (110) surrounded by a cladding (120). The waveguiding core has a first facet (SRF1) and a second facet (SRF2) such that the first facet is arranged to gather the Raman-scattered light from the sample region. The apparatus further comprises a spectrometer (300), and a focusing unit (SF2) that is configured to guide the gathered Raman-scattered light from the second facet to the spectrometer. The spectrometer comprises a detector array (ARR1) that is arranged to measure a spectrum (R_{M}(λ)) of the Raman-scattered light by using time gated detection.

## Description

### FIELD

The present invention relates to a method and an apparatus for measuring a spectrum of Raman-scattered light; and more specifically, to a method and an apparatus for measuring the spectrum of Raman-scattered light using time-gated detection.

### BACKGROUND

Conventional devices for performing spectral analysis may be overly complex to use besides being bulky in size thereby preventing use of such conventional devices to perform the spectral analysis on samples that may be located in areas characterized with tight space constraints.

Practically all materials, including optical elements of a spectral analysis device, may emit light by Raman scattering when illuminated with light, e.g. when the material comprises molecules and/or a crystal structure that has varying degrees of vibrational energy levels. Some molecules may emit light by fluorescence in addition to emitting light by the Raman scattering. For example, organic molecules may emit light by fluorescence and by Raman scattering when illuminated by light. In another example, organic molecules containing conjugated aromatic rings may emit light by fluorescence and by Raman scattering. The fluorescence may disturb measurement of the Raman spectrum. Further, use of separate waveguides for transmitting illuminating light and gathering scattered light may increase system complexity and bulkiness besides incurring additional costs while reducing a coupling efficiency of the transmitted and gathered light.

Therefore, in light of the foregoing discussion, there exists a need to overcome the aforementioned drawbacks to efficiently and reliably perform the spectral analysis of a sample.

### SUMMARY

The present disclosure seeks to provide an apparatus for measuring a spectrum of Raman-scattered light. The present disclosure also seeks to provide a method for measuring a spectrum of Raman-scattered light. The present disclosure seeks to provide a solution to the existing problems associated with conventional devices that are inefficient in that they employ multiple waveguides that may render the conventional device bulky and cost-intensive; and further measurements therefrom are prone to errors owing to the presence of fluorescence in the light received from the sample. An aim of the present disclosure is to provide a solution that overcomes at least partially the problems encountered in prior art by providing an apparatus that is cost-effective and also compact in size for convenient and reliable measurement of a spectrum of Raman-scattered light from a sample region that is located within an area having tight space constraints.

In one aspect, an embodiment of the present disclosure provides an apparatus for measuring a spectrum of Raman-scattered light, the apparatus comprising:
- a light source configured to provide illuminating light pulses;
- an optical probe to guide the illuminating light pulses to a sample region to cause excitation of Raman-scattered light in the sample region, the optical probe comprising a waveguiding core surrounded by a cladding, wherein the waveguiding core has a first facet and a second facet, and wherein the first facet is arranged to gather the Raman-scattered light from the sample region;

- a spectrometer; and
- a focusing unit configured to guide illuminating light pulses to the waveguiding core via the second facet and guide the gathered Raman-scattered light from the second facet to the spectrometer;
wherein the spectrometer comprises a detector array arranged to measure the spectrum of the Raman-scattered light by using time gated detection.

In another aspect, an embodiment of the present disclosure provides a method for measuring a spectrum of Raman-scattered light, wherein the method comprises:
- providing illuminating light pulses;
- guiding the illuminating light pulses to a sample region via a second facet of a waveguiding core of an optical probe;
- causing excitation of Raman-scattered light in the sample region;
- gathering the Raman-scattered light from the sample region at a first facet of the waveguiding core;
- guiding the gathered Raman-scattered light from the first facet, via the second facet, of the waveguiding core to a spectrometer; and
- using time gated detection at the spectrometer to measure the spectrum of the Raman-scattered light from the sample region.

Embodiments of the present disclosure substantially eliminate or at least partially address the aforementioned problems in the prior art, and enable a convenient, reliable, and cost-effective measurement of the spectrum of Raman-scattered light which is devoid of fluorescence prior to performing a spectral analysis on the Raman-scattered light from the sample.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
Fig. 1 is an illustration, by way of example, of an apparatus for measuring a spectrum of Raman-scattered light from a sample region;
Fig. 2 is an illustration, by way of example, of using auxiliary waveguides for guiding illuminating light and for guiding gathered scattered light;
Fig. 3 is an illustration, by way of example, of a spectrometer that comprises a spectral disperser and a detector array;
Fig. 4a is an illustration, by way of example, of a cross section of an optical probe;
Fig. 4b is an illustration, by way of example, of an end of the optical probe;
Fig. 4c is an illustration, by way of example, geometry for illumination and for gathering light when the optical probe is straight;
Fig. 4d is an illustration, by way of example, geometry for illumination and for gathering light when the optical probe is bent;
Fig. 4e is an illustration, by way of example, controlling the geometry for illumination and controlling the geometry for gathering light when the optical probe is bent;
Fig. 5a is an illustration, by way of example, a spectrum of an illuminating light pulse;
Fig. 5b is an illustration, by way of example, of a spectrum of light scattered from the sample material;
Fig. 5c is an illustration, by way of example, of temporal evolution of intensity of an illuminating light pulse, temporal evolution of intensity of scattered light, and temporal response function of a detector pixel;
Fig. 6 is an illustration, by way of example, of temporal evolution of intensity of fluorescence light emitted from the Raman probe;
Fig. 7a is an illustration, by way of example, of a cross sectional of a detector pixel of a detector array and
Fig. 7b is an illustration, by way of example, of the detector array comprising an array of detector pixels.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In one aspect, an embodiment of the present disclosure provides an apparatus for measuring a spectrum of Raman-scattered light, the apparatus comprising:
- a light source configured to provide illuminating light pulses;
- an optical probe to guide the illuminating light pulses to a sample region to cause excitation of Raman-scattered light in the sample region, the optical probe comprising a waveguiding core surrounded by a cladding, wherein the waveguiding core has a first facet and a second facet, and wherein the first facet is arranged to gather the Raman-scattered light from the sample region;
- a spectrometer; and
- a focusing unit configured to guide illuminating light pulses to the waveguiding core via the second facet and guide the gathered Raman-scattered light from the second facet to the spectrometer;
wherein the spectrometer comprises a detector array arranged to measure the spectrum of the Raman-scattered light by using time gated detection.;

In another aspect, an embodiment of the present disclosure provides a method for measuring a spectrum of Raman-scattered light, wherein the method comprises:
- providing illuminating light pulses;
- guiding the illuminating light pulses to a sample region via a second facet of a waveguiding core of an optical probe;
- causing excitation of Raman-scattered light in the sample region;
- gathering the Raman-scattered light from the sample region at a first facet of the waveguiding core;
- guiding the gathered Raman-scattered light from the first facet, via the second facet, of the waveguiding core to a spectrometer; and
- using time gated detection at the spectrometer to measure the spectrum of the Raman-scattered light from the sample region.

The present disclosure discloses the apparatus that is arranged to measure the spectrum of the Raman-scattered light from the sample region by using a single waveguide and by using time gated detection i.e., time resolved detection. Using the time resolved detection together with the optical probe beneficially facilitates ease in performing spectral analysis of the Raman-scattered light from the sample region of a sample that may be located inside a narrow cavity of an object. The apparatus of the present disclosure is compact in size, owing to the use of the single waveguide for illuminating the sample and gathering scattered light from the sample. Moreover, the apparatus facilitates convenient, reliable and error-free measurement of the spectrum of Raman-scattered light from the sample by eliminating, or by at least reducing, fluorescent light in the scattered light received from the sample.

The terms "scattered light", as used herein, may comprise inelastically scattered light and elastically scattered light. The inelastically scattered light may comprise Raman-scattered light and Rayleigh scattered light. Illuminating a sample region with the illuminating pulses may cause emission of Rayleigh scattered light in addition to emission of the Raman scattered light. The inelastically scattered light may also comprise fluorescence light in addition to the Raman scattered light. Light emitted from the sample may comprise light emitted by fluorescence, in addition to light emitted by Raman and Rayleigh scattering.

The sample, or stated particularly - the sample region of the sample material, may comprise molecules that have vibrational and/or rotational energy states. The sample region may have a crystal structure that exhibits multiple vibrational energy states. The Raman scattered light may comprise Stokes-scattered light and anti-Stokes scattered light.

Stokes Raman scattering is an inelastic scattering process where a photon of illuminating light pulse may lose energy into a change of vibrational and/or rotational state of the sample region, i.e. the difference between the energy of the incident photon and the energy of the corresponding scattered photon is positive. That is, the photon of illuminating light pulse may lose energy into a change of vibrational state and manifest into a change of rotational state of the molecules in the sample region.

Anti-Stokes Raman scattering is an inelastic scattering process where a scattered photon may gain energy from a change of vibrational state of the molecules in the sample region. That is, the difference between the energy of the incident photon and the energy of the corresponding scattered photon is negative. The photon of scattered light may gain energy from a change of vibrational state and from a change rotational state of the molecules in the sample region.

A measurement of the spectrum of the Raman-scattered light from the sample region may be used e.g. for providing information about the molecular composition of the sample. The information may be used e.g. for qualitative chemical analysis of the sample, for quantitative chemical analysis of the sample, and/or for analyzing a crystal structure of the sample. An industrial process may be controlled based on the information. The information may be used e.g. for a forensic purpose including, but not limited to, evidence material produced in court.

The sample MX may comprise gas, solid and/or liquid. Further, the sample MX may comprise a heterogeneous mixture. For example, sample MX may comprise a heterogeneous mixture having particles suspended in a liquid or a gas. These particles may be, for example, solid particles or liquid particles. In an exemplary embodiment, the sample may comprise organic and/or inorganic molecules. In another exemplary embodiment, the sample may contain molecules of biological material. In yet another exemplary embodiment, the sample may comprise e.g., a single crystal, a polycrystalline material or an amorphous material. In yet another embodiment, the sample MX may comprise aromatic compounds.

The apparatus comprises the light source to provide illuminating light pulses. The light source may comprise one or more lasers. The illuminating light pulses may be laser pulses. The light pulses may also be called as, for example, excitation light pulses.

In an embodiment, the light source may comprise a passively Q-switched laser. For example, the light source may comprise e.g. a Q-switched Nd:YVO4 laser (neodymium-doped yttrium orthovanadate) or a Q-switched Nd:YAG laser (neodymium-doped yttrium aluminium garnet). Optionally, the light source may comprise e.g. a nonlinear crystal for providing illuminating light from infrared light pulses by frequency conversion. The light source may comprise e.g. a distributed feedback laser or a distributed Bragg reflector laser. Additionally, or optionally, the light source may comprise an optical amplifier that may, in turn, comprise a light-amplifying optical crystal, light-amplifying glass medium, or a light-amplifying optical fiber.

In an embodiment, the repetition rate of the illuminating light pulses LB1 may be in the range of 1 Hz to 100 MHz. In one example, the repetition rate of the laser pulses may be in the range of 10 kHz to 1 MHz. In another example, the repetition rate of the laser pulses may be in the range of 1 MHz to 100 Mhz. The light source may be arranged to generate single pulses according to a trigger signal. The trigger signal may be provided by, for example, a control unit, a detector, a clock or an auxiliary trigger device that may be arranged to send a trigger signal to the light source so as to trigger generating a laser pulse on demand. Optionally, the light source may be arranged to generate a sequence of pulses according to the trigger signal. Further, in an embodiment, a temporal width of the illuminating light pulses may be e.g. in the range of 10 ps to 200 ps. A time constant (τ_{F100}) of fluorescence from the waveguiding core of the optical probe is chosen to be less than a repetition time between two consecutive light pulses of the provided illuminating light pulses. Indeed the repetition rate determines the repetition time and vice versa. If the repetition rate is 1Hz then the repetition time is 1sec i.e. time between two pulses is 1 sec. If the repletion rate is 1M then the repetition time is 1usec (micro second). In other words the duration between the light pulses (LB1) is selected to be longer than the time constant of fluorescence from the core of the probe.

The apparatus comprises the optical probe to guide the illuminating light pulses to the sample region. The sample region may be present on, or inside, a sample material. In this disclosure, the sample material may also be called as e.g. a sample or a sample substance. Further, the sample region may also be called as *e.g.,* a sample volume, or a region of interest.

In an embodiment, the sample material may be a substance present within an industrial process vessel. In another embodiment, the sample material may be a food product. In yet another embodiment, the sample material may be a substance inside at least one of: a human body or an animal body.

In regards to the foregoing embodiment, it may be possible that in some cases, an object may have, or define, a cavity. The object may be e.g. the industrial process vessel having the cavity within which the sample substance e.g., the food product is positioned. The sample material and hence, the sample region of the sample material located inside the object is optically interrogated by inserting the optical probe into the cavity of the object.

The optical probe has an outer diameter that may be in a range of e.g. 20 µm to 1000 µm. Advantageously, the outer diameter of the optical probe may be in the range of 20 µm to 300 µm. Optionally, the optical probe is flexible and a length of the optical probe may be in the range of e.g. 10 mm to 10 m thereby facilitating the optical probe to be inserted into an elongated and/or curved cavity. Further, materials of the optical probe may be selected such that the optical probe may be immersed in e.g. water or another chemical liquid for an extended period of time without damaging the optical probe and/or contaminating the liquid. Therefore, the optical probe is used e.g. as a chemically inert sensor that may be temporarily or permanently immersed inside a material.

In an example, the optical probe is immersed in a molten thermoplastic polymer for monitoring composition of the polymer. In another example, the optical probe is immersed in a composite material in order to monitor a curing state of the composite material during curing of the composite material. In such case, the optical probe may permanently remain inside the cured composite material. In yet another example, the optical probe is used as a disposable sensor that may be immersed into an epoxy resin in order to monitor a composition of the epoxy resin and/or monitor a curing state of the epoxy resin. In yet another example, the optical probe is used for measuring a Raman spectrum of a food product in which the optical probe may be immersed in the food product. In yet another example, the optical probe is an endoscopic probe suitable for performing a biomedical measurement. In such case, the optical probe is a flexible endoscopic probe that is inserted into e.g., a blood vessel, or a cavity, of a human body for identifying a tissue and/or for analyzing a composition of the tissue. Additionally, or optionally, an end of the optical probe is chamfered or rounded to reduce a risk of damaging the sample substance e.g. a blood vessel or another tissue of the human body. In such cases, the optical probe may beneficially comprise, or consist of, bio-compatible materials suitable for in-vivo application. Further, in such cases, the optical probe is sterile and may be stored in a sealed package. Also, the optical probe may be additionally sterilized, if required, e.g., by heating the optical probe upon removal from the sealed package. In such cases, materials for the optical probe may be selected such that the materials of the optical probe allow the optical probe to withstand a temperature higher than e.g., 120°C. In an embodiment, the optical probe is a single use probe i.e., the optical probe may be discarded, or disposed, after a single use.

The optical probe is arranged to illuminate the sample region of the sample with the illuminating light pulses to cause excitation of Raman-scattered light in the sample region. The illuminating light pulses illuminate the sample region to cause inelastic scattering of photons from the sample region. In order to excite Raman-scattered light in the sample region, the optical probe comprises a waveguiding core surrounded by a cladding. In an embodiment, the cladding may be concentrically arranged about the waveguiding core.

Further, materials used for forming respective ones of the waveguiding core and the cladding may be selected such that a refractive index of the waveguiding core is greater than a refractive index of the cladding. The greater refractive index of the waveguiding core, as compared to the refractive index of the cladding, facilitates total internal reflection (TIR) of the illuminating light pulses and the scattered light by the waveguiding core. Due to the total internal reflection of the illuminating light pulses and the scattered light, the illuminating light pulses and the scattered light can be guided effectively by the waveguiding core, i.e., with little or no dispersion of the illuminating light pulses and the scattered light from the waveguiding core. Optionally, a part of the cladding may have a chamfered or rounded edge. The chamfered or rounded edge may also reduce the risk of fracturing the material of the cladding during handling of the optical probe. Also, the chamfered or rounded edge of the cladding prevents any adverse effects on the Raman-scattered light gathering properties of the optical probe because the scattered light is not guided via the cladding, rather the Raman-scattered light is gathered by the waveguiding core of the optical probe i.e., based on the total internal reflection property of the waveguiding core.

The illuminating light and the scattered light may propagate through inorganic optical materials of the apparatus. The illuminating light and the scattered light may excite fluorescence in the inorganic optical materials, e.g. in the materials of the lenses, optical coatings, reflectors, beam splitters, spectrally selective filters, and optical waveguides. The inorganic optical materials are spatially distributed along the path of the illuminating light and/or the scattered light such that fluorescence light, excited at different locations, arrives at the detector array at different times. The apparatus may be arranged to utilize time gated detection to suppress fluorescence light emitted from the optical probe and from other optical components e.g. the focusing unit of the apparatus. Consequently, the disturbing effect of the fluorescence light may be suppressed, eliminated and/or compensated by time gated detection. That is, the total energy of the fluorescence light may be temporally distributed such that the disturbing effect of the fluorescence light may be suppressed, eliminated and/or compensated by time gated detection.

Further, as the materials of the waveguiding core and the cladding are selected so as to reduce the effect of fluorescence light excited inside the optical probe, the optical probe has a low fluorescence quantum yield.

For instance, the waveguiding core may consist essentially of inorganic material, and the cladding may consist essentially of inorganic material. That is, the waveguiding core and the cladding may be substantially free from organic material. In an example, the waveguiding core may comprise, or consist, of silica SiO2, and the cladding may comprise, or consist, of doped silica SiO2. In another example, the waveguiding core may comprise, or consist, of doped silica SiO2 and the cladding may comprise or consist of silica SiO2.

In an embodiment, the optical probe optionally comprises at least one protective coating (hereinafter referred to as "protective layer") surrounding the cladding. For example, the cladding may be surrounded by two protective layers. These protective layers may be concentrically arranged about the cladding. In an example, the protective layer (or layers) may consist of a Fluoropolymer (PTFE, polytetrafluoroethylene). The protective layer may be arranged so as to render the optical probe with an inert outer surface. The term "inert" used herein may be regarded as any substance that is chemically, optically, and thermally inactive i.e., capable of withstanding high operating temperatures, for example, in an industrial process vessel to the propagation of the illuminating light pulses and the scattered light from the sample region when guided by the waveguiding core. According to alternative embodiment the protective coating can be a glass coating, a polymer coating, an aluminum coating or for example a gold coating. Benefit of glass coating is that it can be tolerant to many media such as acids. Benefit of polymer coating is that it does not break easily and it might be more cost efficient to manufacture than glass coating. The gold coating has benefit of being inert to reactions. The aluminum coating is beneficial due to inexpensive costs.

Optionally, the protective layer (or layers) of the optical probe may be opaque so as to block ambient light. However, the optical probe does not need to have an opaque protective layer. Due to the total internal reflection of the waveguiding core, ambient light and/or thermal radiation is not effectively guided to the spectrometer 300, even in a situation where the side of the optical probe would be exposed to intense ambient light and/or thermal radiation. The optical probe may be relatively immune to ambient light and/or thermal radiation.

The waveguiding core of the optical probe has a first facet and a second facet. The first facet and the second facet are arranged at a first opposing end and a second opposing end of the waveguiding core respectively. The first facet may be disposed facing the sample while the second facet is disposed away from the sample. The illuminating light pulses may be coupled into the waveguiding core through the second facet of the waveguiding core. The waveguiding core guides the illuminating light pulses from the second facet to the first facet. The illuminating light pulses exit through the first facet and are incident upon the sample region of the sample.

In an embodiment, respective diameters of the first and second facets are substantially equal to the diameter of the waveguiding core. In a further embodiment, during measurement, the optical probe is positioned such that a distance between the first facet and the sample may be smaller than the diameter of the waveguiding core. In this manner, the scattered light from the sample region may be coupled into the waveguiding core via the first facet, and the scattered light may be guided out of the waveguiding core through the second facet. Therefore, each of the first and second facets of the waveguiding core is used for both - excitation of the sample region and for pick-up of the scattered light.

Moreover, as the first facet of the waveguiding core simultaneously operates as illuminating optics and light gathering optics, the first facet provides a spatially defined illuminated region and gathers scattered light from a spatially defined light gathering region. Using the first facet, for both illuminating the sample and gathering the scattered light from the sample, may therefore ensure that light gathering region and the illuminated region are in optimal alignment with each other. Further, the optical probe may be arranged to deliver illuminating light pulses to the sample region such that the intensity of illuminating light pulses outside the sample region is substantially lower than the intensity of illuminating light inside the sample region. Such use of the first facet for illuminating the sample region and gathering the scattered light from the sample region provides a high quantum yield for Raman scattering from the sample region. The illuminated region may have a substantially conical shape. The shape of the illuminated region may correspond to a truncated cone. The conical boundary of the illuminated region may have a first half cone angle. The light gathering region may also have a substantially conical shape. The conical boundary of the light gathering region may have a second half cone angle greater than the first cone angle of illuminated region. In an embodiment, a time constant of fluorescence from the waveguiding core of the optical probe is longer than 1 ns. In regard to this embodiment, the materials for respective ones of the waveguiding core and the cladding are selected such that a lifetime of the fluorescence light emitted from the waveguiding core and/or from the cladding of the optical probe are e.g. longer than 1 ns. Alternatively, the time constant can be longer than 10 ns, 20 ns, 30 ns, 40 ns, 50ns, 60ns, 70ns, 80 ns, 90 ns, 100ns, 150 ns or 200 ns. Having a time constant with a determined duration is beneficial for when measuring a spectrum using time gate detection, since the time gated detection can be used to "cut of" fluorescence light from the core and the cladding. The time constant duration is preferably longer than time resolution (i.e a temporal width of the illuminating light pulse) of the time gated detection. Further more time between two sequential pulses can be set in a such a way that fluorescence light emitted due to the previous pulse has decayed sufficiently before sending a next pulse.

The apparatus also includes a spectrometer, and a focusing unit. The focusing unit is configured to guide illuminating light pulses from the light source to the waveguiding core, via the second facet of the waveguiding core, and also guide the gathered Raman-scattered light from the second facet of the waveguiding core to the spectrometer. In an embodiment, the focusing unit may comprise one or more first optical focusing elements for focusing illuminating light into the waveguiding core through the second facet of the waveguiding core. For example, the focusing unit may comprise a microscope objective for focusing the illuminating light pulses into the second facet of the waveguiding core. Further, the focusing unit may also comprise of one or more second optical elements that are arranged to gather Raman-scattered light from the second facet of the waveguiding core and guide the gathered Raman-scattered light from the second facet of the waveguiding core to the spectrometer. These second optical focusing elements may include, but are not limited to, lenses, apertures, parabolic reflectors and/or spherical reflectors. In an example, the first and/or the second optical focusing elements of the focusing unit may comprise, inter alia, a beam splitter for separating the path of gathered Raman-scattered light from the path of the incoming illuminating light. Optionally the focusing unit can be a confocal focusing unit.

In a further embodiment, the focusing unit comprises a spectrally selective filter arranged to block wavelengths which are shorter than or equal to the wavelength of the illuminating light pulses. Additionally, or optionally, the spectrally selective filter is arranged to facilitate incidence of a collimated beam of the Raman-scattered light thereon, and to remove one or more spectral components from the scattered light. Therefore, with use of this embodiment, the beam splitter may advantageously be embodied in the form of, for example, a spectrally selective beam splitter for maximizing an efficiency of allowing a predetermined number, or amount, of spectral components, for example, the Raman-scattered light from the scattered light emitted by the sample region upon incidence of the illuminating light pulses.

Alternatively, the illuminating light may be removed from the scattered light e.g. by using one or more separate spectrally selective filters i.e., spectrally selective filters that are distinct from the beam splitter. For example, the focusing unit may comprise a single spectrally selective filter for suppressing light at the wavelength of the illuminating light. This spectrally selective filter may be e.g. a long (wavelength) pass filter, which may block wavelengths which are shorter than or equal to the wavelength of the illuminating light pulses. This spectrally selective filter may be positioned e.g. at a location where the scattered light propagates as a substantially collimated beam from the waveguiding core.

With implementation of the foregoing embodiment, the focusing unit may provide a coaxial optical arrangement whereby excitation of Raman-scattered light, via incidence of the illuminating light pulses on the sample region of the sample, and subsequent pick-up of the Raman-scattered light from the sample region may share the same focus i.e., the illuminating light pulses and the Raman-scattered light may share a common path of travel but in relatively opposite directions. As this coaxial optical arrangement of the excitation i.e., incidence of the illuminating light pulses and pickup i.e., the gathering of the Raman-scattered light is oriented to occur in the same path of travel with the help of the focusing unit, the focusing unit helps to achieve high coupling efficiencies between the sample region and each of the light source and the spectrometer.

Further, if the first and/or second facets inadvertently cause specular Fresnel reflection of the illuminating light pulses backwards towards the spectrometer, these reflected parts of the illuminating light pulses may be suppressed and/or eliminated e.g. by using the first and/or second optical focusing elements of the focusing unit e.g., the beam splitter and/or the spectrally selective filter.

Also, as the illuminating light may be transmitted through several optical components of the first optical focusing elements, these optical components of the first optical focusing elements may add unwanted spectral components to the illuminating light pulses. Further, the illuminating light pulses obtained from the light source may also comprise unwanted spectral components. These unwanted spectral components may be suppressed by providing one or more spectrally selective filters to the first optical focusing elements. Such spectrally selective filters may be positioned e.g. at a location where the illuminating light propagates as a substantially collimated beam. The apparatus may comprise e.g., an aperture to define a width of the collimated beam of the illuminating light pulses.

As the focusing unit may, in some cases, be located outside the cavity e.g., of an industrial process vessel during measurement, it may not be necessary to miniaturize the focusing unit. The first and/or second optical focusing elements of the focusing unit e.g., the filters and/or the beam splitter may have e.g. multilayer coatings to implement spectrally selective properties. Also, the collimated light beams may have a large width (e.g. diameter) so as to enable an optimal amount of spectral filtering. Additionally, or optionally, the focusing unit may comprise of one or more stray light rejection elements and/or beam dumps. Additionally, the focusing unit may be located in a protective enclosure so as to keep the optical components of the focusing unit clean and dry. The focusing unit may be arranged e.g., within the protective enclosure to also facilitate operation at a temperature, which is close to normal room temperature, *e.g.,* in the range of 10°C to 40°C.

In an embodiment, the focusing unit also comprises a spatial filter that is arranged to prevent propagation of at least one of fluorescent or scattered light from the cladding to the spectrometer. In this embodiment, the focusing unit *e.g.,* the second optical focusing elements of the focusing unit may include an aperture that may be arranged to operate as the spatial filter for preventing propagation of any unwanted spectral components of light from the cladding of the optical probe to the spectrometer. Consequently, scattered light and fluorescence light emitted e.g. from the cladding does not disturb the measurement of the spectrum of the Raman-scattered light.

Further, in another embodiment, the focusing unit may additionally comprise of an aperture that is arranged to prevent propagation of fluorescent light from the protective coating to the spectrometer. With implementation of this embodiment, fluorescence light emitted from the protective coating is blocked and hence, does not disturb the measurement of the spectrum of the Raman-scattered light.

The spectrometer comprises the detector array arranged to measure the spectrum of the Raman-scattered light by using time gated detection. The detector array ARR1 may comprise e.g. an array of single photon avalanche diodes (SPAD). The apparatus may therefore be operable to measure the spectral intensity distribution of Raman-scattered light by using an array of single photon avalanche photodiodes (SPAD). Implementation of the SPAD detector as the detector array may beneficially allow e.g. reducing the noise level of the measured Raman-scattered light.

Additionally, or optionally, the spectrometer may comprise a spectral disperser. The spectral disperser may be arranged together with the detector array to measure spectral intensities in a spectral range of the scattered light i.e., the Raman-scattered light. That is, the spectral range may cover one or more spectral ranges of light scattered from the sample, so as to measure the spectrum of the Raman-scattered light from the sample region. The spectral feature may be e.g. a peak or a dip of the spectrum. That is, the measured spectrum may cover the spectral range i.e., the spectrum of the Raman-scattered light from the sample region.

As the apparatus is arranged to determine a measured Raman spectrum, the measured Raman spectrum may be determined from measured signal values obtained from the detector array. The measured spectrum may comprise a plurality of Raman signal values. The measured spectrum of the Raman-scattered light may be an estimate of an actual spectrum of the Raman-scattered light emitted from the sample region of the sample.

In an embodiment, the spectrometer may comprise a spectral disperser. The spectral disperser spatially separates spectral components of the scattered light, and the detector array measures intensity of each of the separated spectral components. The detector array comprises a plurality of detector pixels to detect light at a plurality of different spectral bands. Gathered scattered light may be coupled to the spectrometer e.g. by using the focusing unit, and different spectral components of the scattered light may be spatially separated from each other by using the spectral disperser.

The spectral disperser decomposes the gathered light e.g. into spectral components and directs the separated, or decomposed spectral components of the gathered light to different spatial positions of the detector array. In an example, the spectral disperser may comprise one or more diffraction gratings. In another example, the spectral disperser may comprise one or more diffraction prisms. The detector array comprises an array of detector pixels. In an embodiment, the integration time of each detector pixel of the detector array may be e.g. shorter than 100 ps. Each detector pixel of the detector array may be arranged to measure the spectral intensity of light at a different wavelength. For example, a first spectrally separated component may be directed to a first detector pixel, a second spectrally separated component may be directed to a second detector pixel, a third spectrally separated component may be directed to a third detector pixel, and so on. Consequently, each detector pixel of the detector array may be arranged to measure an intensity of light at a different wavelength band in which the wavelength of each wavelength band may denote a center of said wavelength band. The detector array provides a plurality of measured values indicative of the measured intensities of the corresponding spectral components. That is, each measured value is indicative of spectral intensity of the scattered light at a different wavelength. In a further embodiment, the detector array optionally comprises a memory for storing the measured values. The memory may be called as e.g. a buffer memory. In a further embodiment, the detector array comprises an array of single photon avalanche diodes (SPAD). Using the SPAD detector may allow e.g. reducing the noise level of the measured results.

In an embodiment, the detector array comprises of an array of Complementary Metal Oxide Semiconductor (CMOS) single photon avalanche diodes (SPAD), together abbreviated and referred to as 'CMOS SPAD'. The CMOS SPAD provides for fast response and a spectral sensitivity that matches with the wavelength of the illuminating pulses transmitted by the optical probe. The combination of the optical probe and the CMOS SPAD may be suitable for time-gated measurement of spectrum of Raman-scattered light from the sample region. As the operation of the SPAD detector is time-gated, signal noise and/or the effect of fluorescence is reduced.

In an example, the apparatus may be used for performing Surface Enhanced Raman Spectroscopy on a sample region comprising gold particles. In regards to the foregoing example, the wavelength of the illuminating light pulses may be rendered suitable for exciting the Raman spectrum from the sample region comprising the gold particles. That is, the wavelength of the illuminating light pulses may be in a spectral region e.g. 500 nm to 700 nm where the nanometer-sized gold particles have a high spectral reflectance.

The use of single photon avalanche diodes (SPAD) provides for short response, high sensitivity, and accurate control of time gating. Optionally, the detector array ARR1 may comprise one or more other detectors in place of, or in addition to, the single photon avalanche diodes.

In an exemplary embodiment, the detector array ARR1 comprises an intensified charge-coupled device (ICCD). The intensified charge-coupled device comprises an image intensifier coupled to a CCD device. The image intensifier comprises a photocathode, a micro-channel plate (MCP) and a phosphor screen. When used for measuring Raman scattering, the operation of the intensified charge-coupled device is time-gated to reduce signal noise and/or to reduce the effect of fluorescence.

In another exemplary embodiment, the Raman spectrum may be measured by using an electron multiplying charge coupled device (EMCCD). The EMCCD detector may also be enabled and disabled based on timing of the illuminating light pulses, so as to reduce signal noise and/or so as to reduce the effect of fluorescence.

In yet another exemplary embodiment, the detector array comprises of an array of photomultiplier tubes. For example, the detector array may comprise of two or more photomultiplier tubes. The photomultiplier tubes are arranged to detect single photons. Further, the photomultiplier tubes are arranged to measure spectral intensities in a time gated manner. Accordingly, the apparatus may be arranged to count detected photons by using one or more time-gated counters.

According to one embodiment, spectral data measured by the apparatus may be used for controlling operation of a system. For example, a chemical process or a cell culture process may be controlled based on the Raman spectrum measured by the apparatus.

In an embodiment, the apparatus is arranged to measure a first value indicative of a total intensity at a first time, a second value indicative of fluorescence intensity at a second time. In this embodiment, the apparatus is further arranged to estimate fluorescence intensity at the first time based on at least the measured second value, and determine a Raman signal value from the first value and from the second value by using the estimated fluorescence intensity, as will be explained later herein in conjunction with the drawings.

An embodiment of the present disclosure is also directed towards an optical probe for use in the above-mentioned apparatus.

### DETAILED DESCRPTION OF THE DRAWINGS

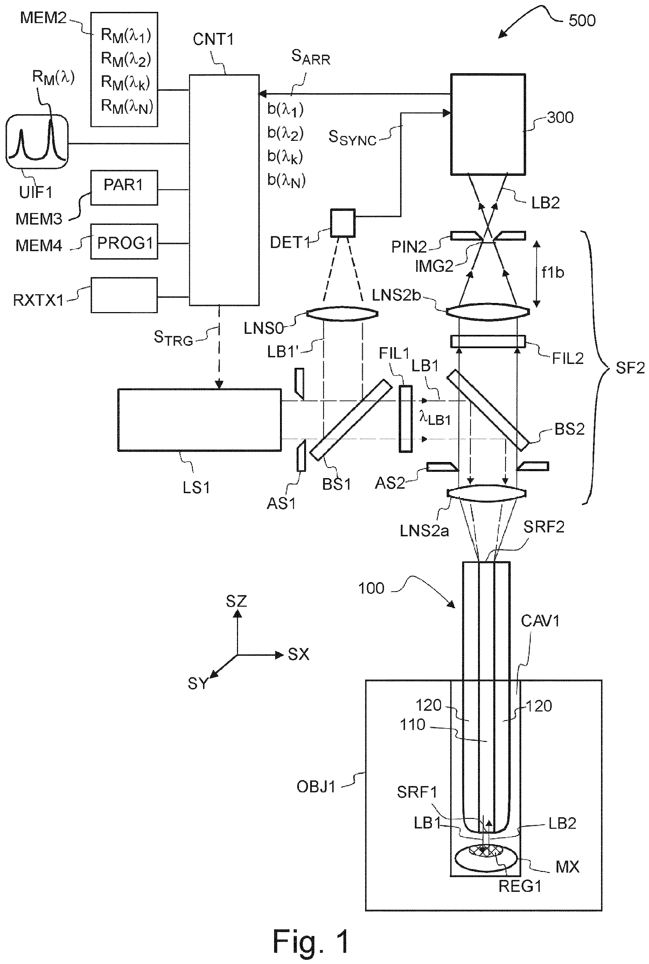

Referring to Fig. 1, the apparatus 500 comprises an illuminating light source LS1 to provide illuminating light pulses LB1 (hereinafter referred to as "illuminating light" and denoted with identical reference alpha-numeral "LB1"). The light source LS1 may be arranged to illuminate a sample region REG1 of a sample MX with the illuminating light pulses LB1. The sample MX is placed within a cavity CAV1 of an object OBJ1. The apparatus 500 further comprises an optical probe 100 that is inserted into the cavity CAV1 of the object OBJ1 for guiding the illuminating light pulses LB1 to the sample MX and for gathering Raman scattered light LB2 from the sample MX out of the cavity CAV1. The optical probe 100 comprises a waveguiding core 110 that is surrounded by a cladding 120. The waveguiding core 110 has a first facet SRF1 that is positioned close to, or in contact with, the sample MX so that the first facet SRF1 faces the sample MX.

The illuminating light LB1 is coupled into the optical probe 100 through a second facet SRF2 of the waveguiding core 110. The optical probe 100 guides the illuminating light LB1 from the second facet SRF2 to the first facet SRF1. The illuminating light LB1 is coupled out of tthe optical probe 100 through the first facet SRF1 of the waveguiding core 110. Scattered light LB2 from the sample region REG1 is coupled into the optical probe 100 through the first facet SRF1 of the waveguiding core 110. The optical probe 100 guides the gathered scattered light LB2 from the first facet SRF1 to the second facet SRF2. The scattered light LB2 is coupled out of the optical probe 100 through the second facet SRF2 of the waveguiding core 110.

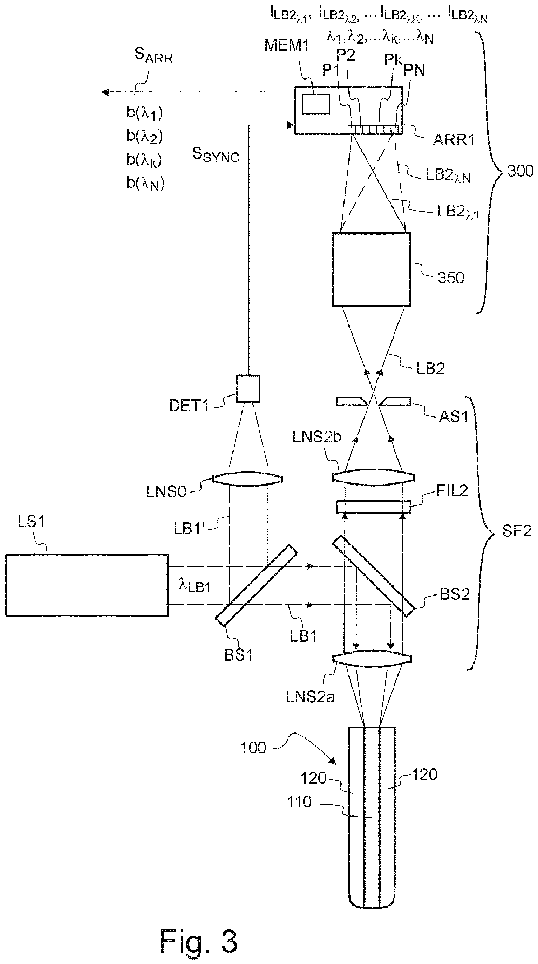

The apparatus 500 comprises a spectrometer 300 for measuring a spectrum of the scattered light LB2. The spectrometer 300 may comprise a spectral disperser 350 and a detector array ARR1 (Refer to Fig. 3). The integration time of detector pixels P₁, P₂, Pₖ,_{.}.. P_{N} of the detector array ARR1 may be e.g. shorter than 100 ps.

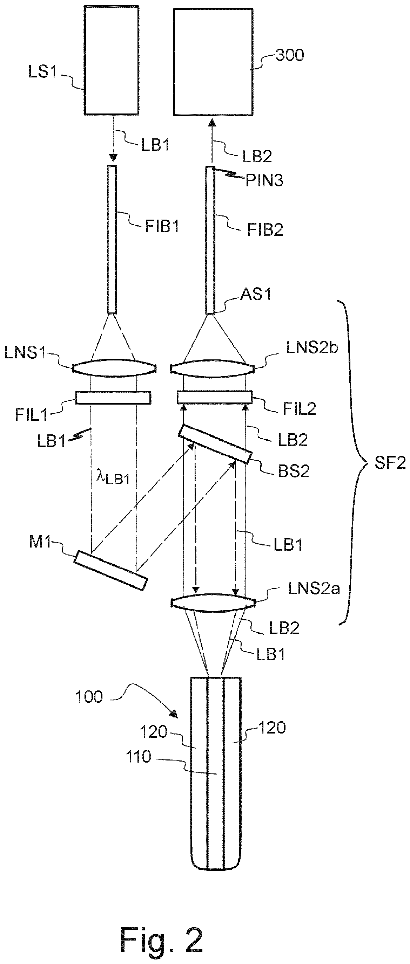

The apparatus 500 may comprise a focusing unit SF2 for coupling illuminating light LB1 into the waveguiding core 110 of the optical probe 100 and for coupling scattered light LB2 from the waveguiding core 110 of the optical probe 100 to the spectrometer 300. The focusing unit SF2 comprises a microscope objective LNS2a for focusing the illuminating light LB1 into the waveguiding core 110. Further, the focusing unit SF2 comprises another microscope objective LNS2b for coupling gathered scattered light LB2 to the spectrometer 300. The microscope objective LNS2b is arranged to focus scattered light LB2 to the spectrometer 300 via an aperture PIN2. In an embodiment as shown in the view of Fig. 2, the aperture PIN2 is omitted and replaced by an end of an optical fiber FIB2. In alternative embodiments, the aperture PIN2 may comprise an entrance slit of the spectrometer itself or a pinhole. The aperture PIN2 may also be called e.g. as a field stop.

Referring again to Fig. 1, the microscope objectives LNS2a, LNS2b together form a substantially sharp image IMG2 of the second facet SRF2 at the distance of the aperture PIN2 (i.e. substantially at the distance f1b from the microscope objective LNS2b). That is, the microscope objective LNS2b has a focal length f1b. The microscope objectives LNS2a, LNS2b and the aperture PIN2 form a combination that operates as a spatial filter. The spatial filter may substantially prevent propagation of light from the cladding 120 of the optical probe 100 to the spectrometer 300.

The apparatus 500 comprises a beam splitter BS2 for separating the path of gathered scattered light LB2 from the path of the incoming illuminating light LB1. As shown, the beam splitter BS2 is arranged to reflect illuminating light LB1 towards the microscope objective LNS2a and to reflect scattered light LB2 towards the microscope objective LNS2b. The beam splitter BS2 may be a spectrally selective beam splitter in order to maximize coupling efficiency.

The focusing unit SF2 may comprise one or more spectrally selective filters FIL2 for suppressing light at a wavelength λ_{LB1} of the illuminating light LB1. In an embodiment, the spectrally selective filter FIL2 is a long (wavelength) pass filter that may block wavelengths of scattered light LB2 shorter than, or equal to, the wavelength λ_{LB1} of the illuminating light LB1 to remove the illuminating light LB1 from the scattered light LB2. As shown in the view of Fig. 1, the filter FIL2 is positioned between the microscope objectives LNS2a and LNS2b. More particularly, the spectrally selective filter FIL2 is positioned between the beam splitter BS2 and the microscope objective LNS2b. That is, the spectrally selective filter FIL2 is positioned at a location where the scattered light LB2 propagates as a substantially collimated beam.

Moreover, in an embodiment herein, if the first and facets SRF1, SRF2 of the waveguiding core 110 cause any specular Fresnel reflection of the illuminating light LB1 backwards towards the spectrometer 300, such reflection of the illuminating light LB1 is suppressed, or eliminated, by the beam splitter BS2 and/or the spectrally selective filter FIL2.

Further, the illuminating light obtained from the light source LS1 may comprise unwanted spectral components. Furthermore, the illuminating light LB1 is transmitted through several optical components that may inadvertently add unwanted spectral components to the illuminating light LB1. As shown, the apparatus 500 further comprises one or more spectrally selective filters FIL1. By using the spectrally selective filter FIL1, these unwanted spectral components may be suppressed, or eliminated from the illuminating light LB1. This spectrally selective filter FIL1 is positioned at a location where the illuminating light LB1 propagates as a substantially collimated beam. Moreover, the apparatus 500 further comprises an aperture AS1 to define a width of the collimated beam LB1.

The apparatus 500 further comprises a synchronization detector DET1 for controlling timing of operation of the detector array ARR1. For sake of simplicity, the synchronization detector DET1 is hereinafter referred to as a timing detector and denoted by identical reference alpha-numeral 'DET1'. The timing detector DET1 is arranged to generate a timing signal S_{SYNC}. The timing signal S_{SYNC} is used for timing an operation of the detector array ARR1 of the spectrometer 300. Further, the timing signal S_{SYNC} is also utilized for synchronizing time gated detection.

Optionally, the apparatus 500 also comprises one or more focusing elements for focusing a portion of light LB1', separated from the illuminating light LB1, to the synchronization detector DET1. Accordingly, as shown in the view of Fig. 1, the apparatus 500 comprises a beam splitter BS1 for separating the portion of light LB1' from the illuminating light LB1. Optionally, the apparatus 500 may further comprise of another microscope objective L_{NS0} for guiding the separated portion of light LB1' from the beam splitter BS1 to the synchronization detector DET1. In response to, and upon receipt of, the portion of light LB1', the synchronization detector DET1 generates the timing signal S_{SYNC}. Accordingly, the signal S_{SYNC} may comprise a timing pulse for controlling the timing of operation of the detector array ARR1. For purposes of simplicity in the present disclosure, the timing signal S_{SYNC} may also be called as a "synchronization signal" and denoted with identical reference 'S_{SYNC}'.

The sample material MX may provide Raman-scattered light, elastically scattered light, and/or fluorescence light when illuminated by the illuminating light pulse LB1. Further, the materials of the optical probe 100 and the materials of the one or more focusing elements may also provide scattered light and fluorescence light when illuminated with the illuminating light LB1. The apparatus 500 is arranged to suppress, eliminate, and/or compensate, the effect of the fluorescence light by time gated detection.

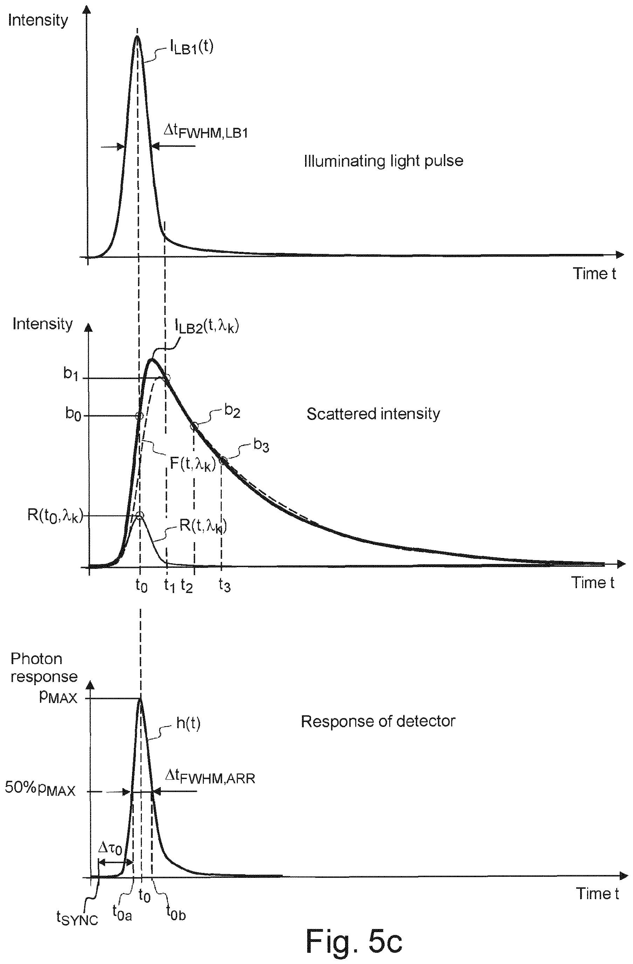

The temporal width Δt_{FWHM,LB1} (refer to Fig. 5c) of the illuminating light pulses LB1 may be relatively small i.e. in the range of 10 ps to 200 ps. The small temporal width Δt_{FWHM,LB1} of the illuminating light pulses LB1 may facilitate measurement of Raman scattered light by time gated detection. The small temporal width Δt_{FWHM,LB1} of the illuminating light pulses LB1 may facilitate suppression of fluorescence in the measurement of the Raman spectrum. That is, time-gated detection may be used in combination with the excitation by the short illuminating light pulses LB1 to facilitate suppression of fluorescence in the measurement of the Raman spectrum.

Moreover, the wavelength λ_{LB1} of the illuminating light pulses LB1 is in the range of 500 nm to 700 nm. The light source LS1 is arranged to generate single pulses according to a trigger signal S_{TRG} that is provided by a control unit CNT1. Optionally, the trigger signal S_{TRG} may be provided by the detector array ARR1, a clock, or another auxiliary trigger device. Optionally, the light source LS1 may be arranged to generate a sequence of pulses according to the trigger signal S_{TRG}. Further, the detector array of the apparatus may be arranged to measure an intensity of the Raman-scattered light LB1 by using time gated detection where an integration time of the detector array may be set with a small temporal resolution e.g. less than, or equal to, 100 ps.

The control unit CNT1 controls operation of the apparatus 500 and/or processes data b(λ₁), b(λ₂), ... b(λₖ), ...b(λ_{N}) obtained from the detector array ARR1. The control unit CNT1 may be configured to determine a measured Raman spectrum P_{M}(λ) from measured values b(λ₁), b(λ₂), ... b(λₖ), ...b(λ_{N}) obtained from the detector array ARR1. Optionally, the apparatus 500 further comprise a memory MEM2 for storing output values P_{M}(λ₁), P_{M}(λ₂), ... R_{M}(λₖ), ...R_{M}(λ_{N}) determined from the measured values b(λ₁), b(λ₂), ... b(λₖ), ...b(λ_{N}). As shown, the measured values b(λ₁), b(λ₂), ... b(λₖ), ...b(λ_{N}) may be communicated from the detector array ARR1 as a signal S_{ARR}. The output values P_{M}(λ₁), P_{M}(λ₂), ... R_{M}(λₖ), ...R_{M}(λ_{N}) specify a measured Raman spectrum P_{M}(λ) of the sample MX. The measured spectrum P_{M}(λ) may represent a Raman spectrum in which fluorescence has been compensated or eliminated.

Optionally, the apparatus 500 may further comprise a memory MEM4 for storing one or more computer programs PROG1. When executed by one or more data processors of the control unit CNT1, the computer program/s PROG1 may cause the apparatus 500 to measure Raman signal values and/or to process Raman signal values. Optionally, or additionally, the apparatus 500 may further comprise a memory MEM3 for storing operating parameters PAR1. The operating parameters PAR1 may specify, among other things, the integration time period for each detector pixel P₁, P₂, Pₖ,_{.}.. P_{N} of the detector array ARR1.

Further, the apparatus 500 comprises a user interface UIF1 for providing information to a user. The user interface UIF1 may also be used for receiving one or more user inputs from the user. The user interface UIF1 may comprise e.g. a display, touch screen and/or a keypad. In an example, the user interface UIF1 may be configured to display a measured Raman spectrum R_{M}(λ) graphically.

Furthermore, the apparatus 500 comprises a communication unit RXTX1 for sending and/or receiving data. The communication unit RXTX1 may be arranged to communicate with e.g. a local area network, an Internet Protocol (IP) address, and/or a mobile communication network. Optionally, the communication unit RXTX1 of the apparatus 500 may also be arranged perform data processing in a distributed manner, for example, by using an Internet server.

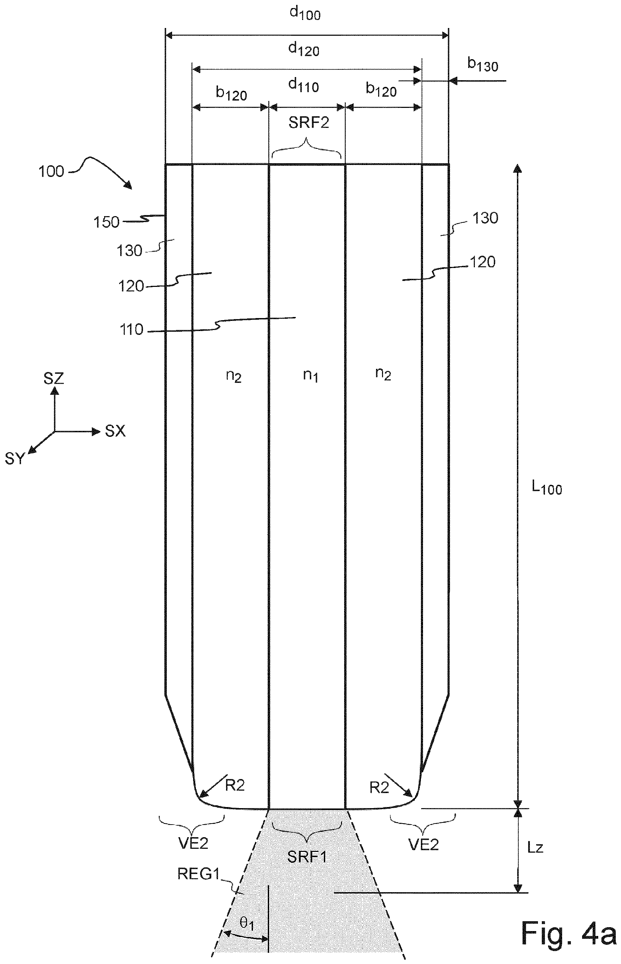

SX, SY and SZ may denote orthogonal directions in the view of Figs. 1, and Figs. 4a-4b respectively.

Referring to Fig. 2, the illuminating light LB1 is coupled into the waveguiding core 110 of the optical probe 100 by using the focusing unit SF2, and the scattered light LB2 is coupled out of the waveguiding core 110 of the optical probe 100 by using the same focusing unit SF2. The apparatus 500 comprises an optical fiber FIB1 for guiding illuminating light LB1 from the light source LS1 to the focusing unit SF2. Optionally, the optical fiber FIB1 may have a large mode area in order to reduce temporal and spectral broadening of a transmitted light pulse LB1.

Further, the apparatus 500 also comprises an optical fiber FIB2 for guiding the scattered light LB2 from the focusing unit SF2 to the spectrometer 300. As the optical fiber FIB2 serves to guide the gathered scattered light LB2 from the focusing unit SF2 to the spectrometer 300, the optical fiber FIB2, or an end of the optical fiber FIB2, may also be arranged to operate as an aperture PIN3. The aperture PIN3 may operate as a part of a spatial filter so as to prevent propagation of light from the cladding 120 of the optical probe 100 to the spectrometer 300.

Optionally, the apparatus 500 further comprises a microscope objective LNS1 for forming a substantially collimated beam of illuminating light LB1. Further, a spectrally selective filter FIL1 is positioned in the collimated beam of illuminating light LB1 to suppress any unwanted and/or potentially disturbing spectral components of the illuminating light LB1. Additionally, or optionally, the apparatus 500 may also comprise one or more reflectors M1 for providing a folded and/or more compact optical set-up.

Further, the apparatus 500 comprises one or more spectrally selective filters FIL1 to spectrally suppress unwanted spectrums of illuminating light and fluorescent light excited in the optical fiber FIB1. The spectrally selective filter FIL1 may be e.g. a short (wavelength) pass filter, which may block wavelengths longer than the wavelength λ_{LB1} of the illuminating light pulses LB1. As shown in the view of Fig. 2, the spectrally selective filter FIL1 is positioned between the optical fiber FIB1 and the reflector M1. More particularly, the filter FIL2 is positioned between the microscope objective LNS1 and the reflector M1. That is, the spectrally selective filter FIL1 is positioned at a location where the illuminating light LB1 propagates as a substantially collimated beam.

Furthermore, the apparatus 500 also comprises one or more spectrally selective filters FIL2 to spectrally suppress fluorescence light excited in the optical probe 100. As shown, the spectrally selective filter FIL2 is positioned between a spectrally selective reflector BS2 and another microscopic objective LNS2b. The spectrally selective filter FIL2 may be e.g. a long (wavelength) pass filter, which may block wavelengths which are shorter than, or equal to, the wavelength λ_{LB1} of the illuminating light pulses LB1.

Moreover, as shown, the illuminating light LB1 is guided from the optical fiber FIB1 to the second facet SRF2 of the optical probe 100, via the microscope objective LNS1, the reflector M1, the spectrally selective reflector BS2, and the microscope objective LNS2a. Further, the scattered light LB2 is guided from the second facet SRF2 to the spectrometer 300 via the microscope objective LNS2a, the spectrally selective reflector BS2, the microscopic objective LNS2b, and the optical fiber FIB2.

Referring to Fig. 3, the spectral disperser 350 spatially separates the gathered light LB2 into different spectral components LB2_{λ1}, LB2_{λ2}, LB2_{λk}, ... LB2_{λN}, and directs the spatially separated spectral components LB2_{λ1}, LB2_{λ2}, LB2_{λk}, ... LB2_{λN} of the scattered light LB2 to different spatial positions of the detector array ARR1. The detector array ARR1 measures intensity of the separated spectral components LB2_{λ1}, LB2_{λ2}, LB2_{λk}, ... LB2_{λN}. The detector array ARR1 comprises a plurality of detector pixels P₁, P₂, Pₖ,_{.}.. P_{N} at the different spatial positions to correspondingly detect and measure the intensity I_{LB2_{λ1}}, I_{LB2_{λ2}}, I_{LB2_{λk}}, ... I_{LB2_{λN}} of light at a plurality of different spectral bands.

The notation 'N' in Fig. 3 denotes the number of spectral bands present in the gathered scattered light LB2. Each detector pixel P₁, P₂, Pₖ,_{.}.. P_{N} of the detector array ARR1 is arranged to measure the spectral intensity of light LB2 at a different wavelength λ₁, λ₂, λₖ, ... λ_{N}. As shown, the spectral component LB2_{λ1} is directed to the detector pixel P₁, the spectral component LB2_{λN} is directed to the detector pixel P_{N}. Each detector pixel P₁, P₂, Pₖ,_{.}.. P_{N} of the detector array 350 may be arranged to measure the intensity I_{LB2_{λ1}}, I_{LB2_{λ2}}, I_{LB2_{λk}}, ... I_{LB2_{λN}} of the gathered scattered light LB2 at a different wavelength band in which the wavelengths λ₁, λ₂, λₖ, ... λ_{N} may denote the centers of said wavelength bands respectively.

The detector array ARR1 provides a plurality of measured values b(λ₁), b(λ₂), b(λₖ), ...b(λ_{N}) indicative of the measured intensities I_{LB_{2λ1}}, I_{LB2_{λ2}}, I_{LB2_{λk}}, ... I_{LB2_{λN}} of the spatially separated spectral components LB2_{λ1}, LB2_{λ2}, LB2_{λk}, ... LB2_{λN}, respectively. Particularly, the detector array ARR1 provides the signal S_{ARR}. The signal S_{ARR} comprises the plurality of measured Raman signal values b(λ₁), b(λ₂), b(λₖ), ...b(λ_{N}). Each measured value b(λ₁), b(λ₂), b(λₖ), ...b(λ_{N}) is indicative of the spectral intensity I_{LB2_{λ1}}, I_{LB2_{λ2}}, I_{LB2_{λk}}, ... I_{LB2_{λN}} of the scattered light LB2 at a different wavelength λ₁, λ₂, λₖ, ... λ_{N}. Optionally, as shown in the view of Fig. 3, the detector array ARR1 also comprises a memory MEM1 e.g. a buffer memory for receiving the signal S_{ARR} and storing the measured values b(λ₁), b(λ₂), ... b(λₖ), ...b(λ_{N}).

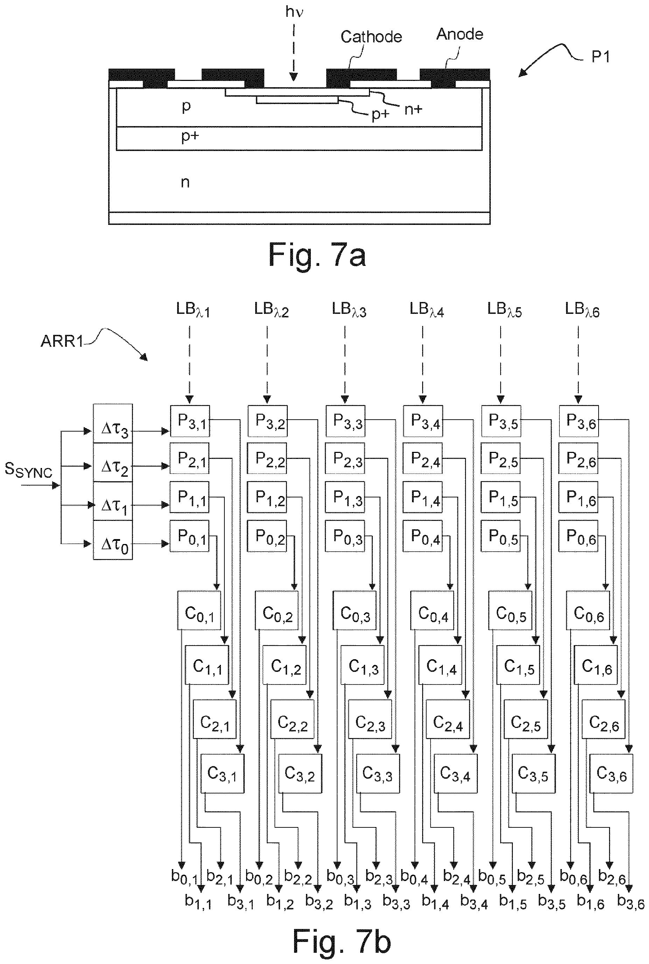

Optionally, the timing signal S_{SYNC} is formed by using the beam splitter BS1 and the synchronization detector DET1. Further, the timing of operation of the detector array ARR1 is controlled based on the timing signal S_{SYNC}. The operation of the detector array ARR1 may be enabled and disabled based on the signal S_{SYNC}. In an embodiment, the detector array ARR1 comprises an array of single photon avalanche diodes (SPAD). In this embodiment, the SPAD of the detector array ARR1 may represent a set of counters, for example, C_{0,1}, C_{1,1}... as shown in the view of Fig. 7b. The operation of these counters C_{0,1}, C_{1,1}... are enabled and disabled based on the timing signal S_{SYNC}.

Referring to Fig. 4a, a cross-section of the optical probe 100 is depicted. As shown, the optical probe 100 comprises the waveguiding core 110 that is concentrically surrounded by the cladding 120. A refractive index n₁ of the waveguiding core 110 is higher than a refractive index n₂ of the cladding 120 so as to enable waveguiding of light LB1 and LB2 in the waveguiding core 110 by total internal reflection (TIR). Optionally, as shown, the optical probe 100 further comprises at least one protective coating 130 surrounding the cladding 120. The protective coating is arranged to render the optical probe with an inert outer surface 150 capable of withstanding a high operating temperature e.g. greater than 120°C.

Optionally, the first facet SRF1 is substantially planar and substantially perpendicular with respect to a centerline XX' of the waveguiding core 110. The first facet SRF1 may be formed by e.g. cleaving, mechanical machining and/or polishing. Similarly, the second facet SRF2 is also substantially planar and substantially perpendicular with respect to the centerline XX' of the waveguiding core 110. The second facet SRF2 may also be formed e.g. by cleaving, by mechanical machining and/or by polishing.

Further, an end of the optical probe 100 may optionally have a chamfered or rounded (circular) edge VE2 so as to facilitate insertion of the optical probe 100 e.g. into the cavity CAV1 of the object OBJ1 (refer Fig. 1). That is, a portion of the cladding 120, located adjacent to the first facet SRF1 of the waveguiding core 110 may have a radius R2. The chamfered or rounded edge VE2 helps reduce the risk of fracturing the material of the cladding 120, and hence, the material of the waveguiding core 110 when maneuvering the optical probe 100 e.g. during insertion into the cavity CAV1 of the object OBJ1.

Optionally, the conical shape of the sample region REG1 may effectively limit a depth of field of the optical probe 100 in the direction SZ. The conical shape of the sample region REG1 may also effectively decrease the intensity of illuminating light with increasing distance Lz from the first facet SRF1 of the waveguiding core 110. Similarly, the efficiency of gathering scattered light LB2 to the first facet SRF1 of the waveguiding core 110 may also decrease with increasing distance Lz of the sample region REG1 from the first facet SRF1. Therefore, the depth of field of the optical probe 100, i.e. the dimension of the sample region REG1 in the direction SZ may be controlled, at least in part, by using short illuminating light pulses and the time-gated detection disclosed later herein.

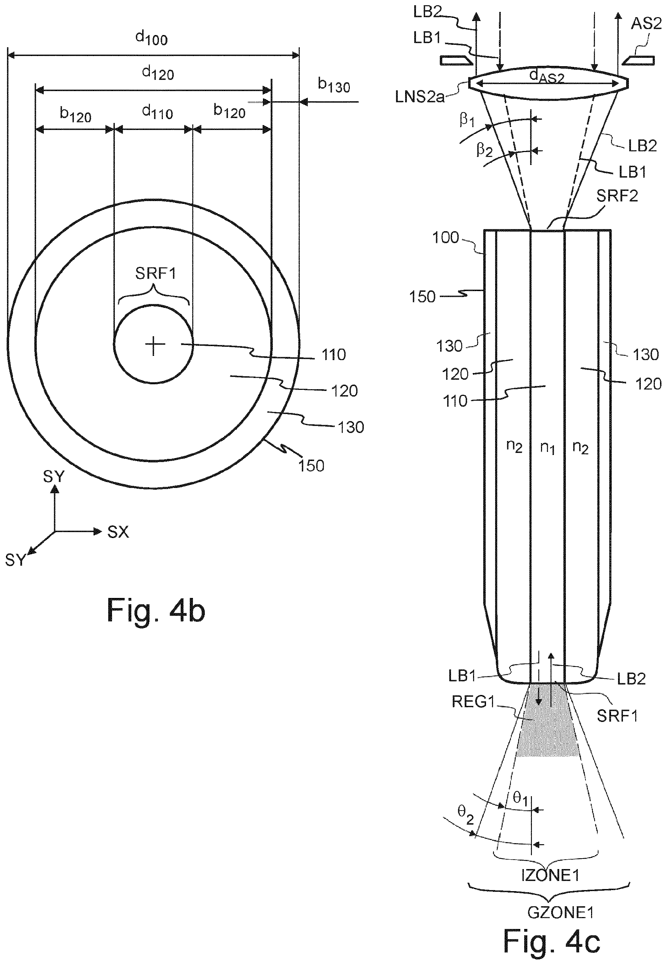

Referring to Fig. 4b, the optical probe 100 has an outer diameter d₁₀₀. The waveguiding core 110 of the optical probe 100 is substantially circular and has a diameter d₁₁₀. The cladding 120 concentrically surrounds the waveguiding core 120. The cladding has an outer diameter d₁₂₀ and a radial dimension b₁₂₀. The protective layer 130 concentrically surrounds the cladding 120 and has a radial dimension b₁₃₀.

Referring to Fig. 4c, the first facet SRF1 may simultaneously operate as illuminating optics and as light gathering optics. The first facet SRF1 may provide a spatially defined illuminated region IZONE1, and the first facet SRF1 may gather scattered light LB2 from a spatially defined light gathering region GZONE1. The sample region REG1 may be defined by the common volume of the illuminated region IZONE1 and the light gathering region GZONE1.

Optionally, the illuminated region IZONE1 has a substantially conical shape. That is, the shape of the illuminated region IZONE1 may correspond to a truncated cone. As shown, the conical boundary of the illuminated region IZONE1 has a half cone angle θ₁. Similarly, the light gathering region GZONE1 may, optionally, be of a substantially conical shape. The conical boundary of the light gathering region GZONE1 has a half cone angle θ₂.

Further, the illuminating light LB1 is coupled into the second facet SRF2 with a maximum half cone angle β₁. The spectrometer 300 is arranged to detect scattered light LB2, which is coupled out of the second facet SRF2 within a half cone angle β₂. The input half cone angle β₁ for illumination corresponds to the output half cone angle θ₁ for illumination, and the output half cone angle β₂ for light gathering corresponds to the input half cone angle θ₂ for light gathering when the waveguiding core 110 is maintained substantially straight during measurement. Additionally, or optionally, the focusing unit SF2 comprises an aperture AS2 for defining the output half cone angle β₂ for light gathering. The input half cone angle β₁ for illumination is also defined by the aperture AS2 and/or by the aperture AS1 (see Fig. 1).

The maximum value of the angles θ₁ and θ₂ may be limited by the numerical aperture NA of the waveguiding core 110. The numerical aperture (NA) of the waveguiding core 110 may be defined by the refractive index n₁ of the waveguiding core 110 and by the refractive index n₂ of the cladding 120 e.g. according to the equation (NA)²=(n₁)²-(n₂)². The maximum value θ_{1,MAX} of the half cone angle θ₁ may depend on the numerical aperture NA e.g. according to the equation NA=n_{MX·}sin(θ_{1,MAX}). The maximum value θ_{2,MAX} of the half cone angle θ₂ may depend on the numerical aperture NA e.g. according to the equation NA= n_{MX}·sin(θ_{2,MAX}). n_{MX} may denote the refractive index of the sample MX that may be in contact with, the first facet SRF1 during measurement. As such, the first facet SRF1 may be in contact with the sample material MX. Additionally, the first facet SRF1 may also operate as a refracting interface between the sample MX and the material (e.g. SiO₂) of the waveguiding core 110. Moreover, a space between the microscope objective LNS2a and the second facet SRF2 may also be filled with a gas or with a material, preferably an inorganic material, having a substantially matching refractive index i.e., ≈n1 as that of the material of the waveguiding core.

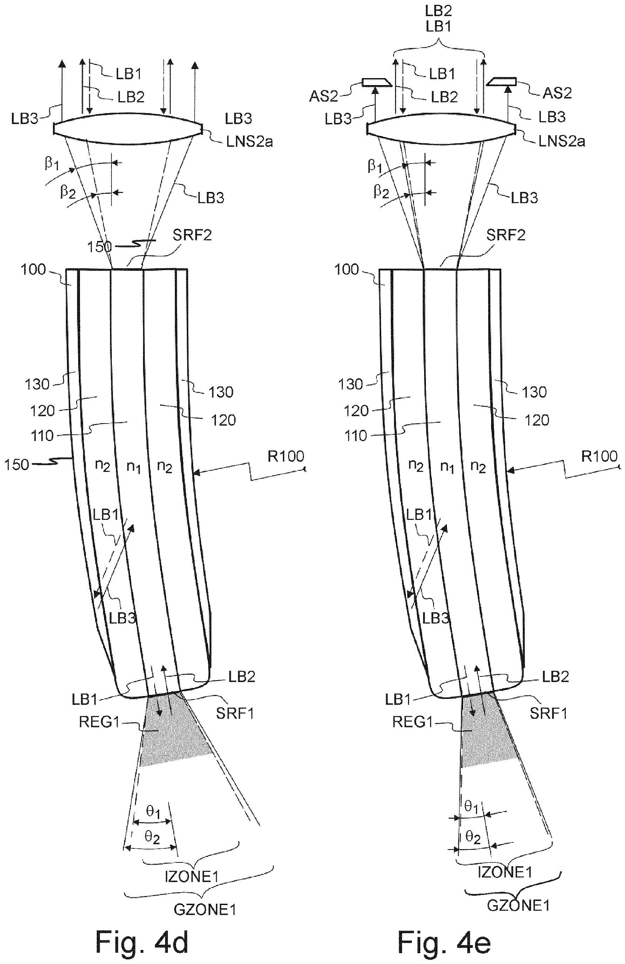

Referring to Figs 4d and 4e, the optical probe 100 may be bent such that the waveguiding core 110 has a radius of curvature R100. The radius of curvature R100 may be e.g. in the range of 100 to 500 times the outer diameter d₁₂₀ of the cladding 120. In case of the bent probe 100, the illuminating light LB1 may leak out of the waveguiding core to the protective coating 130. The illuminating light LB1 may excite fluorescent light LB3 in the protective coating 130. The fluorescent light LB3 emitted from the protective coating 130 may be coupled back into the waveguiding core 110 via the bent section of the waveguiding core 110. The fluorescence light LB3 may disturb measurement of the Raman spectrum of the sample MX if the fluorescence light LB3 emitted from the protective coating 130 is allowed to propagate to the detector array ARR1 of the spectrometer 300.

Referring to Fig. 4e, the apparatus 500 comprises the aperture AS2 as shown. The aperture AS2 is arranged to define the half cone angle θ₁ of the boundary of the illuminated region IZONE1. The apparatus 500 may comprise another aperture e.g. the apertures AS1 (refer Fig. 1) to define the half cone angle θ₂ of the boundary of the light gathering region GZONE1. The first facet SRF1 of the waveguiding core 110 defines the spatial position of the sample region REG1, together with the apertures AS1, AS2 and the focusing unit SF2. As the apertures AS1, AS2 define the boundaries of the regions IZONE1, GZONE1, any light emitted from outside the sample region REG1 is not effectively coupled via the optical probe 100 to the spectrometer 300. That is, the apertures AS2 and/or AS1 are arranged to limit the input half cone angle β₁ of illumination such that the illuminating light LB2 propagating in the waveguiding core 110 is not coupled out of the waveguiding core 110 at the bent section of the optical probe 100.

As the optical probe 100 is flexible, the flexibility of the optical probe 100 facilitates insertion of the optical probe 100 into a cavity having a tortuous path. Consequently, the optical probe 100 may have one or more curved portions during measurement of the Raman spectrum of the sample MX.

Optionally, the aperture AS2 and/or the aperture AS1 may be arranged to limit the input half cone angle β₁ of illumination such that the output half cone angle θ₁ is substantially smaller than the maximum half cone angle θ_{1,MAX} calculated from the equation NA=n_{MX}·sin(θ_{1,MAX}). For example, the aperture AS2 and/or the aperture AS1 may be arranged to limit the input half cone angle β₁ of illumination such that the output half cone angle θ₁ is e.g. smaller than 90% of the maximum half cone angle θ_{1,MAX} calculated from the equation NA=n_{MX·}sin(θ_{1,MAX}).

Further, the aperture AS2 may be arranged to limit the output angle β₂ of light gathering such that propagation of the fluorescence light LB3 from the protective coating 130 to the spectrometer 300 may be substantially prevented. For example, the aperture AS2 may be arranged to limit the output half cone angle β₂ of light gathering such that the input half cone angle θ₂ is e.g. smaller than 90% of the maximum angle θ_{2,MAX} calculated from the equation NA=n_{MX}·sin(θ_{2,MAX}). In one embodiment the output angle can be a function of at least one of: length of the optical probe, refractive indexes of the optical probe.

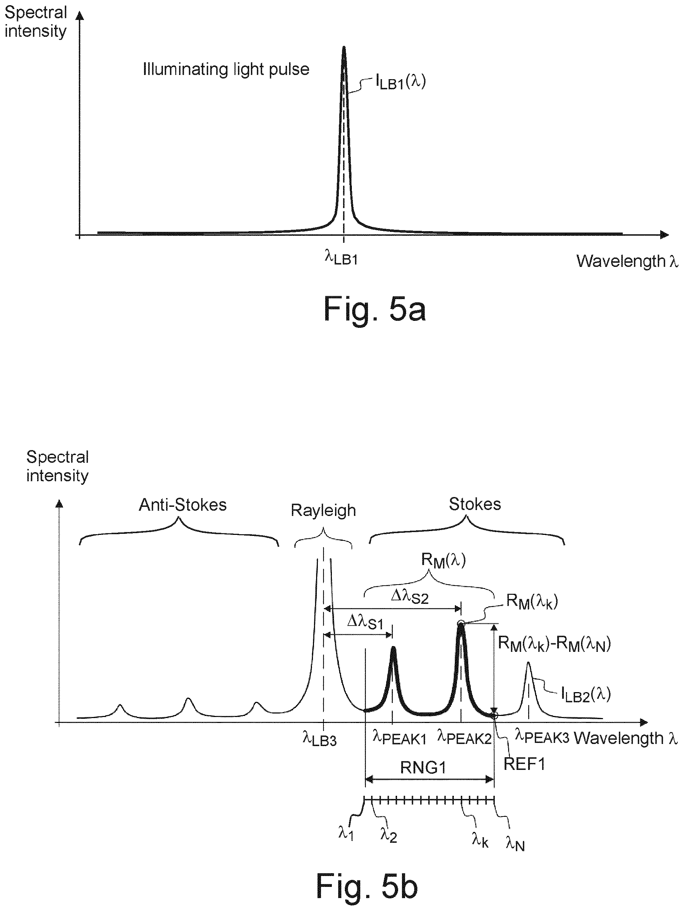

Referring to Fig. 5a, the optical probe 100 may transmit illuminating light pulses LB1 with a spectral intensity distribution I_{LB1}(λ). In particular, an illuminating light pulse LB1 generated at a time to may have a spectral peak, which is located at the wavelength λ_{LB1}.

Fig. 5b shows, by way of example, the spectral intensity distribution I_{LB2}(λ) of scattered light LB2 obtained from the sample MX at the time to, in a situation when the sample MX is illuminated with the illuminating light pulse LB1 of Fig. 5a. Fig. 5b shows only spectral components caused by Raman and Rayleigh scattering. Spectral components caused by fluorescence have been omitted from the spectrum shown in the view of Fig. 5b.

The Rayleigh-scattered light may have high intensity at the wavelength λ_{LB3} of the illuminating light pulse LB1. The stokes Raman-scattered light LB2 obtained from the sample MX may have one or more spectral peaks at the wavelengths λ_{PEAK1}, λ_{PEAK2}, λ_{PEAK3}, which are different from the wavelength λ_{LB1} of the illuminating light pulse LB1. The measured Raman spectrum R_{M}(λ) may comprise e.g. one or more spectral peaks PEAK1, PEAK2. The measured Raman spectrum R_{M}(λ) may comprise e.g. a spectral peak PEAK2 at the wavelength λₖ. The Raman spectrum of the sample MX may comprise a reference point REF1 e.g. at the wavelength λ_{N}. The height R_{M}(λₖ) - R_{M}(λ_{N}) of the spectral peak PEAK2, when compared with the reference point REF1, may be used e.g. for estimating the chemical composition of the sample MX.

The Raman spectrum P_{M}(λ) measured by the apparatus 500 may be compared with reference data e.g. in order to identify a sample MX. A Raman spectrum measured by the apparatus 500 may be compared with reference data e.g. in order to determine a chemical composition of the sample MX. The height and/or position of one or more spectral peaks λ_{PEAK1}, λ_{PEAK2}, λ_{PEAK3} of the measured spectrum R_{M}(λ) may be used for estimating a concentration of a substance in the sample MX. The measured spectrum R_{M}(λ) may be compared with reference spectral data e.g. in order to identify the sample MX. A multivariate chemometric model may be used for qualitative and/or quantitative analysis of the sample MX.

Further, as the spectral disperser 350 and the detector array ARR1 are arranged to measure spectral intensities I_{LB2_{λ1}}, I_{LB2_{λ2}}, I_{LB2_{λk}}, ... I_{LB2_{λN}} in the spectral range RNG1. The spectral range RNG1 may cover one or more spectral features of light scattered from the sample MX, so as to measure the Raman spectrum R_{M}(λ) of the sample MX. The spectral feature may be e.g. a peak or a dip of the spectrum R_{M}(λ).

Fig. 5c exemplarily shows a graphical representation of a temporal evolution of intensity of the illuminating light pulse LB1, a temporal evolution of intensity of scattered light LB2, and a temporal response function of an individual detector pixel P₁, P₂, Pₖ,_{.}.. P_{N} of the detector array ARR1.

The uppermost curve of Fig. 5c exemplarily shows the temporal evolution of the intensity I_{LB1}(t) of illuminating light LB1. An individual illuminating light pulse LB1 may have a temporal width Δt_{FWHM,LB1}. The acronym 'FWHM' represents full-width at half-maximum. The temporal width Δt_{FWHM,LB1} of the pulse LB1 may be e.g. in the range of 10 ps to 200 ps.

The middle curve of Fig. 5c shows the temporal evolution of the spectral intensity R(t,λₖ) of Raman-scattered light LB2, temporal evolution of the spectral intensity F(t,λₖ) of fluorescence light LB3, and temporal evolution of total spectral intensity I_{LB2}(t,λₖ). Temporal evolution of the spectral intensity of the Raman-scattered light LB2 at the wavelength λₖ may be represented by the function R(t,λₖ). Temporal evolution of the spectral intensity of the fluorescence light LB3 at the wavelength λₖ may be represented by the function F(t,λₖ). For the first approximation, the total spectral intensity I_{LB2}(t,λₖ) may equal to the sum R(t,λₖ)+F(t,λₖ).

Referring to the lowermost curve of Fig. 5c, a detector pixel P1 of the detector array ARR1 may be arranged to measure the total spectral intensity I_{LB2}(t,λₖ) at a wavelength λₖ during a short integration time period specified by a timing t. The operation of the detector pixel P1 may be synchronized with the operation of the light source LS1 e.g. by using the synchronization signal S_{SYNC} obtained from the synchronization detector DET1 or the control unit CNT1 (refer Fig. 1). The apparatus 500 may comprise a delay unit (not shown) for controlling and/or adjusting timing of operation of the detector based on the signal S_{SYNC}.

A value b₀ may be measured e.g. during an integration time period comprising a time to. The apparatus 500 may be arranged to operate such that the time t₀ may substantially coincide with the time when the Raman scattered signal reaches a maximum value.

In an embodiment, the measured value b₀ may be directly used as an estimate R_{M}(t,λₖ) for the spectral intensity of the Raman scattered light at the wavelength λₖ.

In an embodiment, a value c₀·b₀ may be used as an estimate R_{M}(t,λₖ) for the spectral intensity of the Raman-scattered light at the wavelength λₖ, wherein c₀ denotes a predetermined coefficient.

The detected intensity I_{LB2}(t,λₖ) of scattered light LB2 may comprise a contribution of fluorescence in addition to the contribution of the Raman scattered light and other scattering mechanisms e.g,. Rayleigh scattered-light. The contribution of the fluorescence may be estimated e.g. from one or more measured intensity values b₁, b₂, b₃. The estimated contribution of the fluorescence may be subsequently eliminated from the measured intensity value b₀.

The apparatus 500 may be arranged to measure Raman spectrum R_{M}(λ) of a sample by using time gated detection i.e. time resolved detection. The total intensity I_{LB2}(t,λ) measured by the detector array ARR1 may comprise the combined effect of Raman-scattered light LB2 and fluorescence light LB3. The apparatus 500 may be configured to measure the first value b₀ at the first time to and the second value b₁ at the second time t₁. The times t₀, t₁ may be selected e.g. such that the Raman-scattered intensity R(t,λ) reaches a peak value at the time to, and such that the Raman-scattered intensity R(t,λ) is e.g. smaller than 10% of the peak value at the time t₁.

The apparatus 500 may be configured to estimate the intensity F(t₀,λ) of fluorescence light at the time to based on one or more values b₁, b₂, b₃ measured after said time to. The apparatus 500 may be configured to estimate the intensity F(t₀,λ) from one or more values b₂, b₃, b₄ e.g. by using a regression function. The apparatus 500 may be configured to estimate the intensity F(t₀,λ) from one or more values b₂, b₃, b₄ e.g. by using an extrapolation function. The apparatus 500 may be configured to calculate the spectral intensity R(t₀,λ) of Raman scattered light based on the value b₀ measured at the time to and based on the estimated spectral intensity F(t₀,λ). Alternatively, the apparatus 500 may be configured to calculate the spectral intensity R(t₀,λ) of Raman scattered light e.g. by subtracting the estimated intensity value F(t₀,λ) from the value b₀.

For example, a value c₀·b₀ - c₁·b₁ may be used as an estimate R_{M}(t,λₖ) for the spectral intensity of the Raman scattered light at the wavelength λₖ, wherein c₀ denotes a first predetermined coefficient, and c₁ denotes a second predetermined coefficient. The coefficients c₀, c₁ may be determined e.g. by calibration tests and/or by simulation carried out before-hand. For example, a value c₀·b₀ - c₁·b₁- c₂·b₂- c₃·b₃ may be used as an estimate R_{M}(t,λₖ) for the spectral intensity of the Raman scattered light at the wavelength λₖ, wherein c₀, c₁, c₂, c₃, denote predetermined coefficients that may be determined e.g. by calibration tests and/or by simulation carried out before-hand. Optionally, determining an estimate R_{M}(t,λₖ) for the spectral intensity of the Raman scattered light at the wavelength λₖ may comprise calculating a linear combination of two or more measured values b₀, b₁, b₂, b₃.

The measured values b₀, b₁, b₂, b₃ obtained from the detector array ARR1 may correspond to a convolution of the total intensity I_{LB2}(t,λₖ) and a temporal response function h(t) of the detector array ARR1. The response function h(t) may be a response function of an individual detector pixel P₁, P₂, Pₖ,_{.}.. P_{N}. Each measured value b₀, b₁, b₂, b₃ may be indicative of the total intensity I_{LB2}(t,λₖ) during an integration time period at the specified spectral position λₖ. For sake of clarity in the present disclosure, the terms "spectral position" may be defined by specifying a wavelength. Also, the term "wavelength" may be regarded as a spectral position.

The integration time period for measuring the values b₀, b₁, b₂, or b₃ may be defined by the temporal width Δt_{FWHM,ARR} of a single detector pixel P1 of the detector array ARR1 and also by the timing of operation of said detector pixel P₁, P₂, Pₖ,_{.}.. P_{N} based on the synchronization signal S_{SYNC}. The accuracy of the time-gated measurement and estimation may be improved when the illuminating light pulse LB1 has a narrow temporal width Δt_{FWHM,LB1} and when the detector pixels P1 of the detector array ARR1 have a narrow temporal width Δt_{FWHM,ARR}.

The detector array ARR1 may comprise a plurality of detector pixels P₁, P₂, Pₖ,_{.}.. P_{N}. An individual detector pixel P1 or a counter associated with the detector pixel may be arranged to provide a signal value b, which may be proportional to the integral of the total spectral intensity I_{LB2}(t,λ) over an integration time period, wherein said signal value b may also be proportional to the integral of the total spectral intensity I_{LB2}(t,λ) over a spectral passband, which has a center at a wavelength λ. The central wavelength λ of the passband associated with a detector pixel may depend on the position of said detector pixel with respect to the spectral disperser 350.

As shown in the middle curve of Fig. 5c, the spectral intensity I_{LB2}(t,λₖ) of the scattered radiation has the value b₀ at the time to and the value b₁ at the time t₁. Further, the intensity I_{LB2}(t,λₖ) has the value b₂ at the time t2. Furthermore, the intensity I_{LB2}(t,λₖ) may have a value b₃ at a time t₃. The values b₀, b₁, b₂, b₃ are obtained by using the detector pixels P₁, P₂, Pₖ,_{.}.. P_{N} of the detector array ARR1. The detector array ARR1 may provide e.g. a one-dimensional array or two-dimensional array of measured values b, which may represent different spectral positions λ₁, λ₂, ...λₖ,...λ_{N}, and/or represent different timings to, t₁, t₂, t₃. The detector array ARR1 may provide e.g. a one-dimensional array [b(λ₁), b(λ₂), ...b(λₖ), ...b(λ_{N})] of measured values b such that the array has a spectral dimension. The detector array ARR1 may provide e.g. a one-dimensional array [b(t₀), b(t₁), b(t₂), b(t₃)] of measured values b such that the array has a temporal dimension. The values b(t₀), b(t₁), b(t₂), b(t₃) may also be denoted by symbols b₀, b₁, b₂, b₃, respectively.

The detector array ARR1 may provide e.g. a two-dimensional array [b(t₀,λ₁), b(t₀,λ₂), ...b(t₀,λₖ), ....b(t₀,λ_{N}), b(t₁,λ₁), b(t₁,λ₂), ...b(t₁,λₖ), ...b(t₁,λ_{N}),...] of measured values b such that the array has a temporal dimension and a spectral dimension. The values [b(t₀,λ₁), b(t₀,λ₂), ... b(t₀,λₖ), b(t₀,λ_{N}), b(t₁,λ₁), b(t₁,λ₂), ...b(t₁,λₖ), ...b(t₁,λ_{N}),....] may also be denoted by symbols b_{0,0}, b_{0,1}, ...b_{0,k}, ...b_{0,N}, b_{1,0}, b_{1,1}, ...b_{1,k}, ... b_{1,N}, ..., respectively.

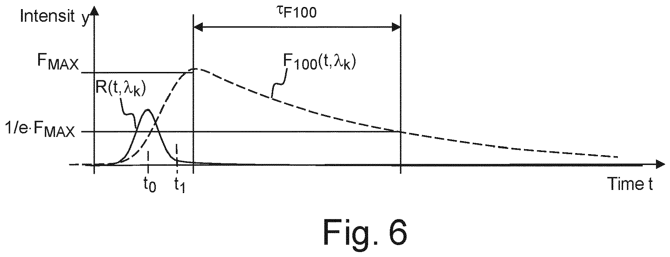

Fig. 6 shows, by way of example, temporal evolution of intensity F₁₀₀(t,λₖ) of fluorescence light emitted from the waveguiding core 110 of the optical probe 100 when illuminated by a single illuminating light pulse LB1. The intensity F₁₀₀(t,λₖ) may have a time constant τ_{F100}. The time constant τ_{F100} may denote e.g. the time during which the fluorescence intensity F₁₀₀(t,λₖ) decreases from a maximum value F_{MAX} to the value 1/e·F_{MAX}. (1/e ≈ 0.368). The materials of the optical probe 100 may be selected such that the time constant τ_{F100} is longer when compared with the temporal width Δt_{FWHM,LB1} of the illuminating light pulse LB1. This way it is possible easier to detect spectrum of the Raman-scattered light using time gated detection as the contribution of the fluorescence signal to the measurement is easier to estimate. In deed the time constant of fluorescence from the core is selected to be longer than a temporal width of the illuminating light pulses.

Referring to Fig. 7a, each detector pixel e.g. the detector pixel P1, or the detector pixel P2 comprises a single photon avalanche diode (SPAD). In particular, a detector pixel P1, P2 may comprise a CMOS SPAD. The acronym 'CMOS' represents a Complementary Metal Oxide Semiconductor. The CMOS SPAD may also be called e.g. as a Geiger mode avalanche photodiode. The SPAD comprises a p-n junction, which may be reverse-biased such that a single charge carrier injected into the depletion layer of the p-n junction may trigger a self-sustaining avalanche of charge carriers. The single charge carrier may be generated by a photon hv. When the avalanche begins, the current through the p-n junction may rapidly rise so that the leading edge of the avalanche current pulse may mark an arrival time of the detected photon hv. The rapid change of the current through the p-n junction may be detected by a suitable electronic circuit. The current through the p-n junction may continue until the avalanche is quenched. The SPAD may be used together with a quenching circuit that may be arranged to quench the avalanche current caused by a photon.

The apparatus 500 may comprise an electronic unit (not shown), which may be arranged to detect a rapid chance of the avalanche current and also to quench the avalanche current. Thus, the single photon avalanche diode may comprise a reverse-biased p-n junction, wherein each photon hv impinging on an active area of the single photon avalanche diode may be arranged to cause a detectable current pulse with a probability, which is substantially greater than zero. The detection probability may also be called e.g. as a quantum efficiency. The quantum efficiency of the single photon avalanche diode may be greater than e.g. 20% for photons hv in a predetermined wavelength range. In one example, the quantum efficiency of the single photon avalanche diode may be greater than 20% for photons in the predetermined wavelength range of 400 nm to 900 nm. In another example, the quantum efficiency of the single photon avalanche diode may be greater than 30% for photons in the predetermined wavelength range of 440 nm to 680 nm. In yet another example, the quantum efficiency of a single photon avalanche diode may be greater than 40% for photons in the range of 470 nm to 630 nm.

In an embodiment, the detector pixels P₁, P₂, Pₖ,_{.}.. P_{N} of the detector array ARR1 may be arranged as a one-dimensional spatial array in order to measure the Raman spectrum R_{M}(λ), which corresponds to a single integration period. The detector array ARR1 may comprise e.g. 1x32, 1x64, 1x128, 1x256, 1x512, 1x1024, 1x2048, ... detector pixels.

Optionally, detector pixels of the detector array ARR1 may be arranged as a two-dimensional spatial array in order to measure a first spectrum, which corresponds to a first integration period, and to measure a second spectrum, which corresponds to a second integration period. The number of pixels in the spatial dimension may be e.g. in the range of 1 to 100, and the number of pixels in the spectral dimension may be e.g. in the range of 10 to 10000. The detector array ARR1 may comprise e.g. 2x32, 2x64, 2x128, 2x256, 2x512, 2x1024, 2x2048, .... detector pixels P1. The detector array ARR1 may comprise e.g. 4x32, 4x64, 4x128, 4x256, 4x512, 4x1024, 4x2048, ... detector pixels P1.

When using the detector array ARR1, the detector array ARR1 may comprise a set of counters arranged to count pulses provide by the photodiodes. The operation of said set of counters may be enabled and disabled based on the timing signal S_{SYNC}.

The lowermost curve of Fig. 5c shows a temporal response function h(t) of a single photon avalanche diode. The response function h(t) may also be called e.g. as the single photon response of the single photon avalanche diode. The temporal width Δt_{FWHM} of the response function h(t) may be e.g. shorter than or equal to 60 ps. For example, the temporal width Δt_{FWHM} of the response function h(t) may be e.g. in the range of 35 ps to 50 ps. For example, the temporal width Δt_{FWHM} may be substantially equal to 40 ps. The apparatus 500 may comprise a delay unit for controlling a time delay Δτ₀. The time delay Δτ₀ may denote e.g. a difference between the time t₀ₐ of the rising edge of the temporal response function h(t) and the time t_{SYNC} of arrival of a timing pulse S_{SYNC} to the detector array ARR1.

The temporal response function h(t) may also be interpreted to represent the probability of detecting a photon per unit time. The probability of detecting a photon per unit time may reach a maximum value p_{MAX}. The probability of detecting a photon per unit time may reach 50% of the maximum value p_{MAX} at the times t₀ₐ, t_{0b}. For example, the integrated area of the response function h(t) between the times t₀ₐ, t_{0b} may correspond to a probability of e.g. 10% for detecting a photon, which impinges on a predetermined detector pixel P1 during the time period between the times t₀ₐ, t_{0b}. The temporal width Δt_{FWHM} of the response function h(t) may be determined e.g. by calculating the difference t_{0b}-t₀ₐ. The function h(t) may represent the temporal response function h(t) of a single photon avalanche diode regarding a single detection period. That is, the function h(t) may represent the probability of detecting a photon per unit time when counting photons for a single detection period. The function h(t) may represent the probability of detecting a photon per unit time when counting photons of scattered light LB2 emitted from the sample region REG1 during a time period at a given wavelength λ.

The apparatus 500 may comprise one or more counting units for counting the number of photons detected by a detector pixel P1. The apparatus 500 may comprise one or more counting units for counting the number of photons detected by a second detector pixel P2. In an embodiment, the detector pixels P1, P2 and the counting units may be implemented on the same (semiconductor) substrate. The counting units may be implemented e.g. by means of a computer program running on one or more data processors and/or by means of electronic circuits implemented on a substrate.

The apparatus 500 may comprise a counting unit, which may be arranged to count the number of photons detected by a detector pixel P1 when the sample is illuminated by several light pulses LB1. The counting unit may be arranged to change a counter value each time when a single detector pixel P1 detects a photon during a predetermined (integration) time period. The time period may be precisely timed with respect to the synchronization pulse S_{SYNC}. A counting unit may be arranged to change a counter value each time when the single detector pixel P1 detects a photon during a predetermined time period.

For each illuminating light pulse LB1, the single detector pixel P1 may detect only one photon. The operating parameters of the apparatus 500 may be selected such that the probability of detecting a photon may be in the range of e.g. 0.01% to 10% when the sample region REG1 is illuminated by a single light pulse LB1.

N_{P1} may denote the number of photons detected by a detector pixel P1. N_{LB1} may denote the number of light pulses LB1. The light source LS1 may be arranged to provide N_{LB1} illuminating light pulses LB1 during a time period T_{SEQ}. The length of the time period T_{SEQ} may be e.g. in the range of 1 ms to 1000 s, and the number N_{LB1} may be e.g. in the range of 10² to 10⁶. The intensity of the illuminating light pulses, the duration of the light pulses and the light gathering efficiency of the gathering optics 20 may be selected such that the ratio N_{P1}/N_{LB1} may be e.g. in the range of 0.01% to 10%.

If the ratio N_{P1}/N_{LB1} is too low, a total time needed to measure the Raman spectrum P_{M}(λ) may be excessively long. If the ratio N_{P1}/N_{LB1} is too high, the measured values b₁, b₂, b₃ may not be proportional to the intensity of the scattered light LB2, i.e. the relationship between the intensity of the scattered light LB2 and the measured values b₁, b₂, b₃ become nonlinear. Advantageously, the ratio N_{P1}/N_{LB1} may therefore be e.g. smaller than or equal to 1 %.

Referring to Fig. 7b, the detector array ARR1 may comprise a pixel P_{0,1} for detecting a photon at a wavelength λ₁ during a first integration time period, a pixel P_{1,1} for detecting a photon at a wavelength λ₁ during a second integration time period, a pixel P_{2,1} for detecting a photon at a wavelength λ₁ during a third time period, and a pixel P_{3,1} for detecting a photon at a wavelength λ₁ during a fourth time period. The detector array ARR1 may comprise a counting unit C_{0,1} arranged to provide a measured signal b_{0,1} by counting photons detected by the pixel P_{0,1}. The detector array ARR1 may comprise a counting unit C_{1,1} arranged to provide a signal b_{1,1} by counting photons detected by the pixel P_{1,1}. The detector array ARR1 may comprise a counting unit C_{2,1} arranged to provide a signal b_{2,1} by counting photons detected by the pixel P_{2,1}. The detector array ARR1 may comprise a counting unit C_{3,1} arranged to provide a signal b_{3,1} by counting photons detected by the pixel P_{3,1}.

The detector array ARR1 may comprise a pixel P_{0,2} for detecting a photon at a wavelength λ₂ during the first integration time period, a pixel P_{1,2} for detecting a photon at a wavelength λ₂ during the second integration time period, a pixel P_{2,2} for detecting a photon at a wavelength λ₂ during the third integration time period, and a pixel P_{3,2} for detecting a photon at a wavelength λ₂ during a fourth integration time period. The detector array ARR1 may comprise a counting unit C_{0,2} arranged to provide a signal b_{0,2} by counting photons detected by the pixel P_{0,2}. The detector array ARR1 may comprise a counting unit C_{1,2} arranged to provide a signal b_{1,2} by counting photons detected by the pixel P_{1,2}. The detector array ARR1 may comprise a counting unit C_{2,2} arranged to provide a signal b_{2,2} by counting photons detected by the pixel P_{2,2}. The detector array ARR1 may comprise a counting unit C_{3,2} arranged to provide a signal b_{3,2} by counting photons detected by the pixel P_{3,2}.

The detector array ARR1 may comprise pixels P and counters C for providing measured signals b in a corresponding way at further wavelengths λ₃, λ₄, λ₅, λ₆, ... λₖ, λₖ₊₁, ... λ_{N}. The detector array ARR1 may comprising the pixels P and counters C are arranged e.g. for measuring the spectral intensity at the different wavelength bands having the central wavelengths λ₁, λ₂, ...λₖ, λₖ₊₁, ... λ_{N}. The detector array ARR1 may comprise pixels P for measuring the spectral intensity e.g. at 1024 adjacent wavelength bands.

The timing of the first, second, third and fourth integration time periods with respect to the synchronization pulse S_{SYNC} may be controlled e.g. by delays Δτ₀, Δτ₁, Δτ₂, Δτ₃. That is, the apparatus 500, or the detector array ARR1 in particular, may comprise delay units for providing the delays Δτ₀, Δτ₁, Δτ₂, Δτ₃.

In an embodiment, the signals b_{0,1}, b_{1,1}, b_{2,1}, b_{3,1} may also be provided by using a single photon avalanche diode (e.g. P_{0,1}). In an embodiment, the signals b_{0,1}, b_{1,1}, b_{2,1}, b_{3,1} may also be provided by using a single photon avalanche diode (e.g. P_{0,1}) and by using the counting units C_{0,1}, C_{1,1}, C_{2,1}, C_{3,1}. For example, the counting unit C_{0,1} may be arranged to provide a signal value b_{0,1} by counting photons detected by the single photon avalanche diode P_{0,1} during the first integration time period, the counting unit C_{1,1} may be arranged to provide a signal value b_{1,1} by counting photons detected by the single photon avalanche diode P₀,₁ during the second integration time period, the counting unit C_{2,1} may be arranged to provide a signal value b_{2,1} by counting photons detected by the single photon avalanche diode P_{0,1} during the third integration time period, and the counting unit C_{3,1} may be arranged to provide a signal value b_{3,1} by counting photons detected by the single photon avalanche diode P_{0,1} during the third integration time period. For example, the detector array ARR1 or the control unit CNT1 may comprise a memory MEM1 for storing values of the signals b_{0,1}, b_{1,1}, b_{2,1}, b_{3,1}.

The signals b_{0,2}, b_{1,2}, b_{2,2}, b_{3,2} may also be provided by using the single photon avalanche diode (e.g. P_{0,2}). The signals b_{0,3}, b_{1,3}, b_{2,3}, b_{3,3} may also be provided by using the single photon avalanche diode (e.g. P_{0,3}). The signals b_{0,4}, b_{1,4}, b_{2,4}, b_{3,4} may also be provided by using the single photon avalanche diode (e.g. P_{0,4}). The signals b_{0,5}, b_{1,5}, b_{2,5}, b_{3,5} may also be provided by using the single photon avalanche diode (e.g. P_{0,5}). The signals b_{0,6}, b_{1,6}, b_{2,6}, b_{3,6} may also be provided by using the single photon avalanche diode (e.g. P_{0,6}).

The detector array ARR1 may be arranged to operate such that the counters C may be enabled and disabled based on the synchronization signal S_{SYNC}. The integration of the optical signal with the detector array ARR1 may be enabled and disabled with a short time response resulting in no penalty from detector read noise which may be the case when using e.g. a conventional CCD detector. Using the detector array ARR1 may thus allow splitting a total measurement time period into a plurality of integration times. Measured data obtained during said (different) integration times may be sorted into separate groups (e.g. in different memory areas). For example, a first intensity value representing a first integration time may be stored in a first memory area, and a second intensity value representing a second subsequent integration time may be stored in a second memory area.

In an embodiment, the apparatus 500 may comprise two or more probes (not shown), and an optical multiplexer, wherein the two or more optical probes may be used in a time-multiplexed manner. The two or more optical probes of the apparatus 500 may be arranged to monitor e.g. two or more sample locations using a single spectrometer e.g. the spectrometer 300.

For the person skilled in the art, it will be clear that modifications and variations of the devices and the methods according to the present invention are perceivable. The figures are schematic. The particular embodiments described above with reference to the accompanying drawings are illustrative only and not meant to limit the scope of the invention, which is defined by the appended claims.

## Claims

1. An apparatus (500) for measuring a spectrum (R_{M}(λ)) of Raman-scattered light (LB2), the apparatus comprising:
- a light source (LS1) configured to provide illuminating light pulses (LB1);
- an optical probe (100) to guide the illuminating light pulses to a sample region (REG1) to cause excitation of Raman-scattered light in the sample region, the optical probe comprising a waveguiding core (110) surrounded by a cladding (120), wherein the waveguiding core has a first facet (SRF1) and a second facet (SRF2), and wherein the first facet is arranged to gather the Raman-scattered light from the sample region;
- a spectrometer (300); and
- a focusing unit (SF2) configured to guide illuminating light pulses to the waveguiding core via the second facet and guide the gathered Raman-scattered light from the second facet to the spectrometer;
wherein the spectrometer comprises a detector array (ARR1) arranged to measure a spectrum (R_{M}(λ)) of the Raman-scattered light by using time gated detection.

2. The apparatus (500) of claim 1, wherein the focusing unit (SF2) comprises a spatial filter arranged to prevent propagation of at least one of fluorescent or scattered light from the cladding (120) to the spectrometer (300).

3. The apparatus (500) of claim 2, wherein the focusing unit (SF2) comprises a spectrally selective filter (FIL2) arranged to block wavelengths which are shorter than or equal to the wavelength (λ_{LB1}) of the illuminating light pulses (LB1).

4. The apparatus (500) according to any one of the preceding claims, wherein the spectrally selective filter (FIL2) is arranged to facilitate incidence of a collimated beam of the Raman-scattered light (LB2) thereon, and to remove one or more spectral components from the scattered light.

5. The apparatus (500) according to any one of the preceding claims, wherein a time constant (τ_{F100}) of fluorescence from the core (110) of the probe (100) is longer than 1 ns.

6. The apparatus (500) according to claim 5 wherein the time constant of fluorescence from the core is selected to be longer than a temporal width of the illuminating light pulses.

7. The apparatus (500) according to claim 5 or 6 wherein a duration between the light pulses (LB1) is selected to be longer than the time constant of fluorescence from the core of the probe.

8. The apparatus (500) according to any one of the preceding claims, wherein the optical probe (100) comprises at least one protective coating (130) surrounding the cladding (120) to render the optical probe with an inert outer surface (150), and wherein the focusing unit (SF2) comprises an aperture (AS2).

9. The apparatus (500) according to any of the preceding claims, wherein the spectrometer (300) further comprises a spectral disperser (350) configured to spatially separate spectral components (LB2_{λ1}, LB2λ₂, LB2_{λk}, LB2_{λN}) of the scattered light; and the detector array (ARR1) is configured to receive the spatially separate spectral components (LB2_{λ1}, LB2λ₂, LB2_{λk}, LB2_{λN}) of the scattered light from the spectral disperser and measure an intensity (I_{LB2_{λ1}}, I_{LB2_{λ2}}, I_{LB2_{λk}}, I_{LB2_{λN}}) of each of the spatially separate spectral components (LB2_{λ1}, LB2λ₂, LB2_{λk}, LB2_{λN}).

10. The apparatus (500) according to any of the preceding claims, wherein the apparatus (500) is arranged to:
- measure a first value (bo) indicative of a total intensity (I_{LB2}(t,λ)) at a first time (to);
- measure a second value (b₁) indicative of fluorescence intensity (F(t,λ)) at a second time (t₁);
- estimate fluorescence intensity (F(t₀,λ)) at the first time (to) based on at least the measured second value (b₁); and
- determine a Raman signal value (R_{M}(t₀,λ)) from the first value and from the second value by using the estimated fluorescence intensity.

11. An apparatus according to any of the preceding claims, wherein the aperture (AS2) is arranged to limit an output angle to prevent propagation of the fluorescence light (LB4) from the protective coating to the spectrometer (300).

12. An apparatus according to claim 10, wherein the output angle is a function of at least one of: length of the optical probe, refractive indexes of the optical probe.

13. An optical probe (100) for use in an apparatus (500) according to any of the claims 1-12, to guide illuminating light pulses (LB1) to a sample region (REG1) to cause excitation of Raman-scattered light (LB2) in the sample region (REG1), the optical probe (100) comprising a waveguiding core (110) surrounded by a cladding (120), wherein the waveguiding core has a first facet (SRF1) and a second facet (SRF2), and wherein the first facet is arranged to gather the Raman-scattered light from the sample region.

14. A method for measuring a spectrum (R_{M}(t₀,λ)) of Raman-scattered light (LB2), wherein the method comprises:
- providing illuminating light pulses (LB1);
- guiding the illuminating light pulses (LB1) to a sample region (REG1) via a second facet (SRF2) of a waveguiding core (110) of an optical probe (100) ;
- causing excitation of Raman-scattered light (LB2) in the sample region (REG1) ;
- gathering the Raman-scattered light (LB2) from the sample region (REG1) at a first facet (SRF1) of the waveguiding core (110);
- guiding the gathered Raman-scattered light (LB2) from the first facet, via the second facet, of the waveguiding core to a spectrometer (300); and
- using time gated detection at the spectrometer (300) to measure the spectrum (R_{M}(λ)) of the Raman-scattered light (LB2) from the sample region (REG1).

15. The method according to claim 14, further comprising:
- inserting the first facet (SRF1) of the optical probe (100) into the sample region (REG1) on at least one of: a sample material (MX) or a cavity (CAV1) within the sample material (MX); and
- measuring the spectrum (R_{M}(λ)) of the Raman-scattered light (LB2) gathered from the sample material (MX).

16. The method according to claim 14 or 15, wherein the sample material (MX) is a substance present within an industrial process vessel (OBJ1), and the method comprises arranging the first facet (SRF1) of the optical probe (100) in the industrial process vessel (OBJ1), and measuring the spectrum (R_{M}(λ)) of the Raman-scattered light (LB2) gathered from the substance present within an industrial process vessel.

17. The method according to any of the claims 14 to 16, wherein the sample material (MX) is a food product, and the method comprises inserting the optical probe (100) into the food product, and measuring the spectrum (R_{M}(λ)) of the Raman-scattered light (LB2) gathered from the food product.

18. The method according to any of the claim 14 to 17, wherein the sample material (MX) is a substance inside at least one of: a human body or an animal body, and the method comprises inserting the optical probe (100) into at least one of: the human body or the animal body, and measuring the spectrum (R_{M}(λ)) of the Raman-scattered light (LB2) gathered from the substance inside at least one of: the human body or the animal body.

19. The method according to any of the claims 15 to 18, comprising separating a first optical probe (100) from the focusing unit (SF2), and attaching a second optical probe (100) to the focusing unit (SF2).
